# NEUE EUROPÄISCHE PATENTSCHRIFT

(11) **EP 3 048 097 B2**
(45) Veröffentlichungstag und Bekanntmachung des Hinweises auf die Entscheidung über den Einspruch: **01.12.2021**
(45) Hinweis auf die Patenterteilung: 24.04.2019
(21) Anmeldenummer: 16152159.6
(22) Anmeldetag: 21.01.2016
(51) Int. Cl.: C07D 401/14

(54) **VERFAHREN ZUR HERSTELLUNG EINER N-METHYLSUBSTITUIERTEN TRIACETONAMINVERBINDUNG**
METHOD FOR PRODUCING AN N-METHYL SUBSTITUTED TRIACETONE AMINE COMPOUND
PROCEDE DE FABRICATION D'UNE LIAISON DE TRIACETONAMINE N-METHYL-SUBSTITUE

(30) Priorität: 22.01.2015 EP 15152068
(43) Veröffentlichungstag der Anmeldung: 27.07.2016
(73) Patentinhaber: Evonik Operations GmbH, 45128 Essen (DE)
(72) Erfinder: NIEMEYER, Jochen, 45128 Essen (DE); WILLY, Benjamin, 40211 Düsseldorf (DE); NISSEN, Felix, 48301 Nottuln (DE); NEUMANN, Manfred, 45770 Marl (DE); KREILKAMP, Guenter, 45768 Marl (DE); SCHERING, Sabine, 46514 Schermbeck (DE)
(74) Vertreter: Evonik Patent Association

(56) Entgegenhaltungen:
- EP-A1- 0 729 947
- EP-A2- 0 302 020
- EP-A2- 0 375 333
- DE-A1- 2 545 695
- DE-A1- 2 618 580
- JP-A- S 559 019
- US-A- 3 974 127
- US-A- 4 049 647
- US-A- 4 316 837
- US-A- 4 757 144
- US-A- 6 046 304
- Houben-Weyl: Methoden der organischen Chemie, vol. 11, no. 1, 1957, page 602-603, 641-647,

## Beschreibung

### Verfahren zur Herstellung einer N-methylsubstituierten Triacetonaminverbindung

Die vorliegende Erfindung beschreibt ein Verfahren zur Herstellung einer *N*-methylsubstituierten Triacetonaminverbindung (Triacetonamin = "TAA"). Die vorliegende Erfindung beschreibt dabei insbesondere ein Verfahren zur Methylierung des im Ring befindlichen Stickstoffes, wie beispielsweise in der Reaktionsgleichung <1> dargestellt (R ist dabei zum Beispiel ein Alkylrest, ein Alkoxyrest, ein Aminrest oder auch eine OH-Gruppe):

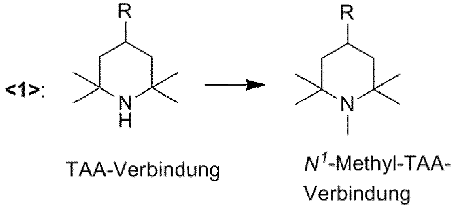

### Hintergrund der Erfindung

Methylierte Derivate des 2,2,6,6-Tetramethylpiperidins sind besonders in der Anwendung als *"hindered amine light stabilizers"* von besonderer Bedeutung. Durch die Methylierung wird eine Anwendung auch unter sauren Bedingungen ermöglicht. Kommerzielle Produkte, welche über *N*-methylierte 2,2,6,6-Tetramethylpiperidin-Gruppen verfügen, sind z. B. Tinuvin^{®} 292 [eine Mischung aus 1-(Methyl)-8-(1,2,2,6,6-pentamethyl-4-piperidinyl)-sebacat und *Bis*-(1,2,2,6,6-pentamethyl-4-piperidinyl)-sebacat] oder Cyasorb^{®} UV-3529 (CAS NUMBER 193098-40-7).

Im Stand der Technik sind verschiedene Verfahren zur Methylierung von Aminen beschrieben.

So beschreiben z. B. Kopka, I. E., et al., J. Org. Chem. 1980, 45, 4616 - 4622 und Minatti, A., et al., J. Org. Chem. 2007, 72, 9253 - 9258 die Umsetzung von Aminen mit Methylhalogeniden. Diese ist schematisch in der Reaktionsgleichung **<2>** dargestellt (R ist dabei ein Rest, wie er in Bezug auf die Reaktionsgleichung **<1>** definiert ist):

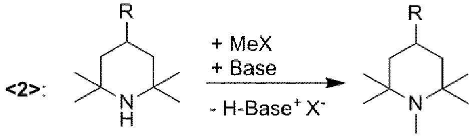

Nachteilig an dem in Reaktionsgleichung **<2>** dargestellten Verfahren ist dabei, dass zur Freisetzung des Produktes neben dem Methylhalogenid mindestens ein Äquivalent einer geeigneten Base eingesetzt werden muss. Dies führt zudem zur Bildung der entsprechenden Salze, welche dann als Abfallprodukt entstehen. Problematisch ist zudem, dass eine selektive Alkylierung zum tertiären Amin im Allgemeinen nicht möglich ist, da auch eine Überalkylierung zum entsprechenden quartären Ammoniumsalz stattfinden kann.

Ein weiteres im Stand der Technik beschriebenes Verfahren ist die Eschweiler-Clarke-Reaktion (z. B. Lutz, W. B., et al., J. Org. Chem. 1962, 27, 1695 - 1703; EP 0 729 947 A1; WO 2004/072035 A1; WO 2005/123679 A2). Bei diesem Verfahren wird das Amin in Gegenwart von Ameisensäure mit Formaldehyd umgesetzt. Die Ameisensäure fungiert dabei als Reduktionsmittel und wird in CO₂ überführt. Damit die Reaktion abläuft, wird zudem im Allgemeinen ein Äquivalent Base benötigt. Die Reaktion ist schematisch in der folgenden Reaktionsgleichung **<3>** wiedergegeben (R ist dabei ein Rest, wie er in Bezug auf die Reaktionsgleichung **<1>** definiert ist):

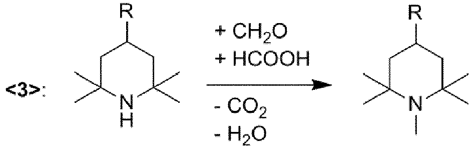

Nachteilig ist hier die nötige Verwendung von Ameisensäure. Zudem führt die Verwendung der Base wiederum zur Generierung eines entsprechenden Abfallstromes.

Eine weitere im Stand der Technik (z. B. WO 2004/089913 A1, WO 2008/101979 A1) beschriebene Möglichkeit zur *N*-Methylierung stellt die Umsetzung mit Formaldehyd in Anwesenheit von Borhydriden dar (z. B. Natriumborhydrid). Die Reaktion ist schematisch in der folgenden Reaktionsgleichung **<4>** wiedergegeben (R ist dabei ein Rest, wie er in Bezug auf die Reaktionsgleichung **<1>** definiert ist):

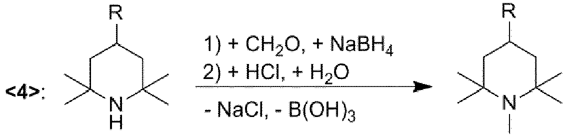

Nachteilig ist hier die nötige Verwendung der Borhydride. Bei der Aufarbeitung entstehen große Mengen an Borsäure oder Borsäurederivaten als Abfallstrom.

Die Aufgabe der vorliegenden Erfindung bestand deshalb darin, ein Verfahren zur Herstellung einer *N*-methylsubstituierten Triacetonaminverbindung zur Verfügung stellen, welches die vorstehend genannten Nachteile nicht aufweist.

Es wurde nun überraschend ein Verfahren gefunden, welches die vorbeschriebene Aufgabe löst.

### Beschreibung der Erfindung

Die vorliegende Erfindung betrifft in einem ersten Aspekt ein Verfahren gemäß den folgenden Punkten 1.1 bis 1.7.
1.1 Verfahren zur Herstellung einer *N*-methylsubstituierten Triacetonaminverbindung,
   dadurch gekennzeichnet, dass man mindestens eine Triacetonaminverbindung **(I)** mit Formaldehyd unter reduktiven Bedingungen umsetzt,
   wobei die Triacetonaminverbindung **(I)** ausgewählt ist aus der Gruppe bestehend aus den chemischen Strukturen **(I-F), (I-G), (I-H)** mit 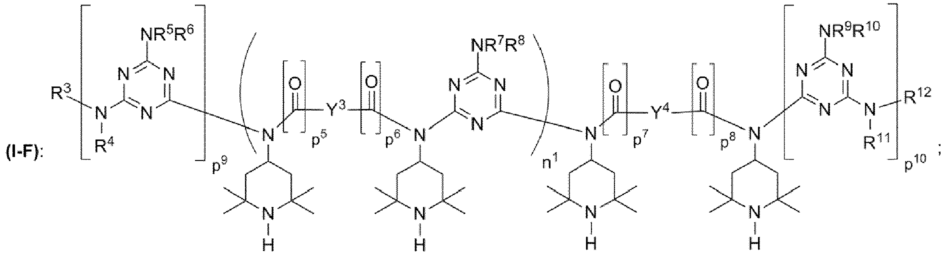 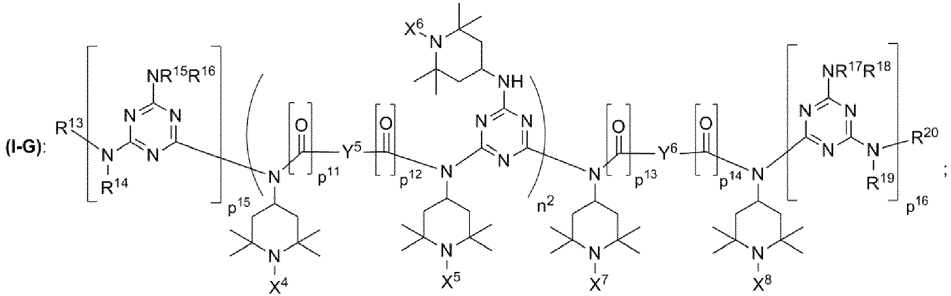 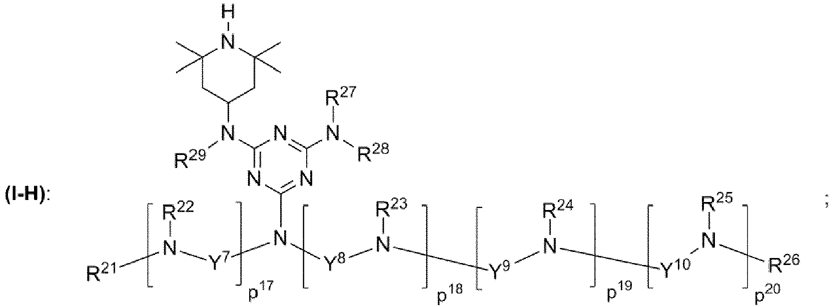
   wobei n¹, n² unabhängig voneinander jeweils eine ganze Zahl aus dem Bereich 1 bis 20 ist;
   wobei p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰ unabhängig voneinander jeweils 0 oder 1 ist;
   wobei X⁴ = X⁵ = X⁶ = X⁷ = X⁸ = Wasserstoff;
   wobei Y³, Y⁴, Y⁵, Y⁶, Y⁷, Y⁸, Y⁹, Y¹⁰ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche
         mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus (i), (ii) mit 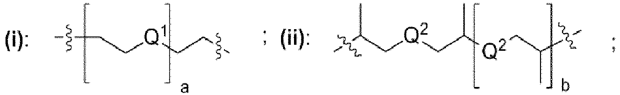 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y³ auch eine direkte Bindung sein kann, falls mindestens einer von p⁵ und p⁶ den Wert 1 aufweist,
   und wobei Y⁴ auch eine direkte Bindung sein kann, falls mindestens einer von p⁷ und p⁸ den Wert 1 aufweist,
   und wobei Y⁵ auch eine direkte Bindung sein kann, falls mindestens einer von p¹¹ und p¹² den Wert 1 aufweist,
   und wobei Y⁶ auch eine direkte Bindung sein kann, falls mindestens einer von p¹³ und p¹⁴ den Wert 1 aufweist;
   wobei die Reste R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, R³⁰ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus (iii), **(iv), (v), (vi), (vii), (viii)**, **(ix) mit** 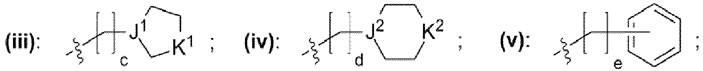 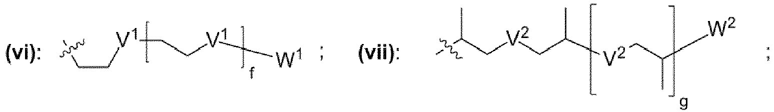 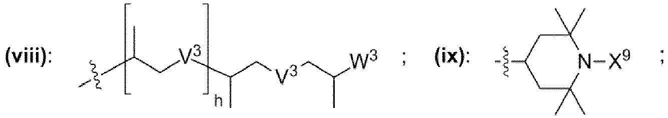 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ = Wasserstoff,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R⁷, R⁸ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x**) sein kann,
   und wobei, wenn p¹⁰ = 1, -NR¹¹R¹² auch ein Rest der chemischen Struktur **(x)** sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur (x) wie folgt definiert ist: 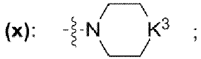
   wobei K³ ausgewählt ist aus der Gruppe bestehend aus -O-, -S-, -NH-, -N(CH₃)-, -N(CH₂CH₃)-, wobei K³ bevorzugt -O- ist;
   wobei die Reste R²¹, R²², R²³, R²⁴, R²⁵, R²⁶ unabhängig voneinander jeweils ausgewählt sind aus der Gruppe bestehend aus
      Wasserstoff, unverzweigte oder verzweigte Alkylgruppe mit 1 bis 30 Kohlenstoffatomen,
      einer Gruppe mit der chemischen Struktur (xi) mit 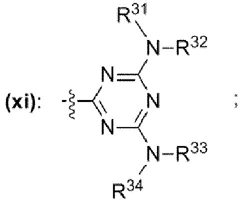
      wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(xii), (xiii), (xiv), (xv), (xvi), (xvii), (xviii)** mit 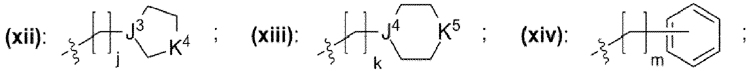 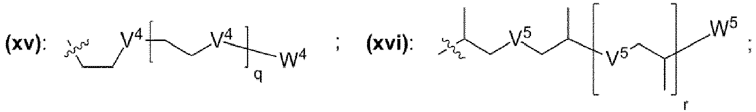 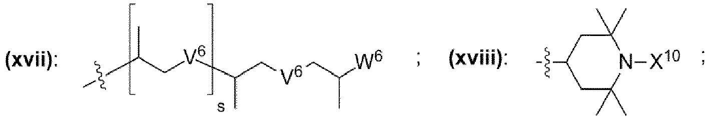 aufweist,
         wobei J³, J⁴ unabhängig voneinander jeweils ausgewählt aus der Gruppe bestehend aus CH, N sind,
         wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist,
         wobei X¹⁰ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
         wobei in den chemischen Strukturen **(xii), (xiii), (xiv), (xv), (xvi), (xvii), (xviii)** mindestens ein an ein Kohlenstoffatom gebundener Wasserstoffrest durch einen Rest ausgewählt aus der Gruppe bestehend
            aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 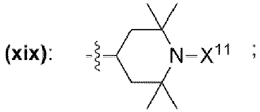
   sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
   und wobei man reduktive Bedingungen dadurch einstellt, dass man die mindestens eine Triacetonaminverbindung **(I)** mit Formaldehyd in Gegenwart von Wasserstoff und in Gegenwart eines Trägerkatalysators umsetzt, wobei der Trägerkatalysator mindestens ein Metall **M** aufweist, wobei das Metall **M** ausgewählt ist aus der Gruppe bestehend aus Cr, Mo, Mn, Ni, Pd, Pt, Ru, Rh.
1.2 Verfahren nach Punkt 1.1, wobei p⁵ = p⁶ = p⁷ = p⁸ = p¹¹ = p¹² = p¹³ = p¹⁴ = 0 ist und wobei p⁹, p¹⁰, p¹⁵, p¹⁶, p¹⁷, p¹⁸, p¹⁹, p²⁰ unabhängig voneinander jeweils 0 oder 1 ist.
1.3 Verfahren nach Punkt 1.1 oder 1.2, wobei
   Y³, Y⁴, Y⁵, Y⁶, Y⁷, Y⁸, Y⁹, Y¹⁰ unabhängig voneinander jeweils ausgewählt ist aus der Gruppe bestehend aus
   unverzweigte oder verzweigte Alkylengruppe mit 1 bis 30 Kohlenstoffatomen, zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
1.4 Verfahren nach einem oder mehreren der Punkte 1.1 bis 1.3, wobei die Reste R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur (ix) mit 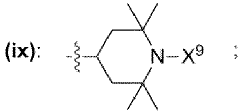 aufweist,
      wobei X⁹ = Wasserstoff;
   wobei die Reste R⁷, R⁸ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur (x) sein kann,
   und wobei, wenn p¹⁰ = 1, -NR¹¹R¹² auch ein Rest der chemischen Struktur (x) sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 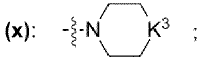 wobei K³ ausgewählt ist aus der Gruppe bestehend aus -O-, -S-, -NH-, -N(CH₃)-, -N(CH₂CH₃)-, wobei K³ bevorzugt -O- ist;
   wobei die Reste R²¹, R²², R²³, R²⁴, R²⁵, R²⁶ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen,
   einer Gruppe mit der chemischen Struktur **(xi)** mit 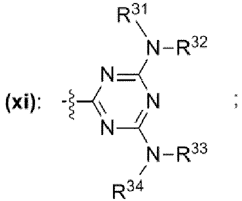 wobei die Reste R²⁷, R²⁸, R²⁹, R³¹, R³², R³³, R³⁴ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(xviii)** mit 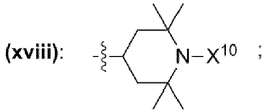 aufweist,
      wobei X¹⁰ = Wasserstoff,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 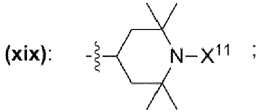 sein können, wobei X¹¹ = Wasserstoff.
1.5 Verfahren nach einem oder mehreren der Punkte 1.1 bis 1.4, wobei Formaldehyd als Gas, als wässrige Lösung oder als Feststoff eingesetzt wird, bevorzugt als wässrige Lösung oder als Feststoff, noch bevorzugter als wässrigeLösung.
1.6 Verfahren nach einem oder mehreren der Punkte 1.1 bis 1.5, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungs-mittel, Ether, halogenierte Lösungsmittel, Amide, Thioverbindungen, Carbonsäuren, Alkohole, Wasser.
1.7 Verfahren nach einem oder mehreren der Punkte 1.1 bis 1.6, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

Die vorliegende Erfindung betrifft in einem zweiten Aspekt ein Verfahren gemäß den folgenden Punkten 2.1 bis 2.8.
2.1 Verfahren zur Herstellung einer *N*-methylsubstituierten Triacetonaminverbindung, dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Struktur **(I-F)** mit 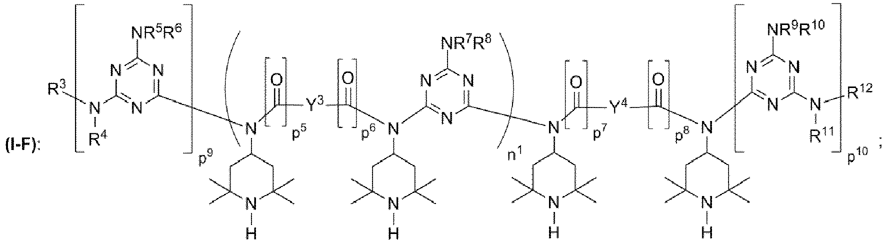
   wobei n¹ eine ganze Zahl aus dem Bereich 1 bis 20 ist;
   wobei p⁵, p⁶, p⁷, p⁸, p⁹, p¹⁰ unabhängig voneinander jeweils 0 oder 1 ist;
   wobei Y³, Y⁴ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche
         mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus (i), (ii) mit 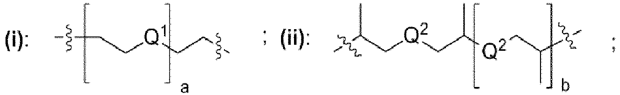 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   und wobei Y³ auch eine direkte Bindung sein kann, falls mindestens einer von p⁵ und p⁶ den Wert 1 aufweist,
   und wobei Y⁴ auch eine direkte Bindung sein kann, falls mindestens einer von p⁷ und p⁸ den Wert 1 aufweist,
   wobei die Reste R³, R⁴, R⁵, R⁶, R⁹, R¹⁰, R¹¹, R¹² unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v), (vi), (vii), (viii)**, **(ix)** mit 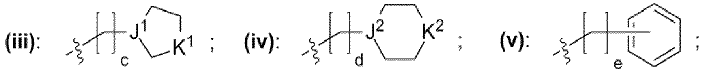 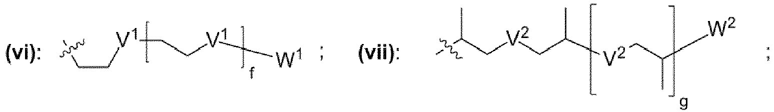 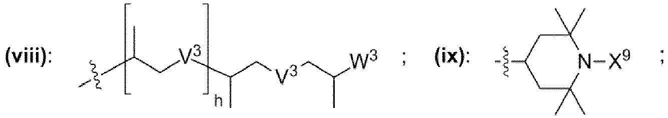 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
   wobei die Reste R⁷, R⁸ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,
   und wobei, wenn p¹⁰ = 1, -NR¹¹R¹² auch ein Rest der chemischen Struktur **(x)** sein kann,
   und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 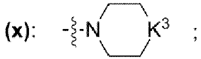 wobei K³ ausgewählt ist aus der Gruppe bestehend aus -O-, -S-, -NH-, -N(CH₃)-, -N(CH₂CH₃)-, wobei K³ bevorzugt -O- ist,
   und wobei man reduktive Bedingungen dadurch einstellt, dass man die mindestens eine Triacetonaminverbindung **(I)** mit Formaldehyd in Gegenwart von Wasserstoff und in Gegenwart eines Trägerkatalysators umsetzt, wobei der Trägerkatalysator mindestens ein Metall **M** aufweist, wobei das Metall **M** ausgewählt ist aus der Gruppe bestehend aus Cr, Mo, Mn, Ni, Pd, Pt, Ru, Rh.
2.2 Verfahren nach Punkt 2.1, wobei p⁵ = p⁶ = p⁷ = p⁸ = 0 ist und wobei p⁹, p¹⁰ unabhängig voneinander jeweils 0 oder 1, ist; bevorzugt ist dabei p⁵ = p⁶ = p⁷ = p⁸ = 0 ist und mindestens einer von p⁹, p¹⁰ ist 1 (wobei logischerweise für den Fall, in dem nur einer vor p⁹, p¹⁰ = 1 ist, der andere von p⁹, p¹⁰ = 0 ist); noch bevorzugter ist p⁵ = p⁶ = p⁷ =p⁸ = 0 und p⁹ = p¹⁰ = 1.
2.3 Verfahren nach Punkt 2.1 oder 2.2, wobei Y³, Y⁴ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
2.4 Verfahren nach einem oder mehreren der Punkte 2.1 bis 2.3, wobei die Reste R³, R⁴, R⁵, R⁶, R⁹, R¹⁰, R¹¹, R¹² unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 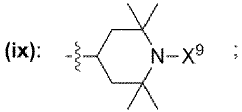 aufweist,
      wobei X⁹ = Wasserstoff;
   wobei die Reste R⁷, R⁸ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei, wenn p⁹ = 1, -NR³R⁴ auch ein Rest der chemischen Struktur **(x)** sein kann,und wobei, wenn p¹⁰ = 1, -NR¹¹R¹² auch ein Rest der chemischen Struktur **(x)** sein kann,und wobei die Reste -NR⁵R⁶, -NR⁷R⁸, -NR⁹R¹⁰ unabhängig voneinander jeweils auch ein Rest der chemischen Struktur **(x)** sein können,
   wobei die chemische Struktur **(x)** wie folgt definiert ist: 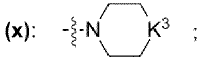 wobei K³ ausgewählt ist aus der Gruppe bestehend aus -O-, -S-, -NH-, -N(CH₃)-, -N(CH₂CH₃)-, wobei K³ bevorzugt -O- ist.
2.5 Verfahren nach einem oder mehreren der Punkte 2.1 bis 2.4, wobei die Reste R³, R⁴, R⁵, R⁶, R⁹, R¹⁰, R¹¹, R¹² unabhängig voneinander jeweils ausgewählt sind aus der Gruppe bestehend aus
   Wasserstoff,
   unverzweigte oder verzweigte Alkylgruppe mit 1 bis 12 Kohlenstoffatomen,
   einem Rest, der eine chemischen Struktur **(ix)** mit 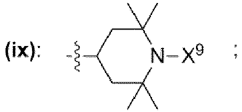 aufweist,
      wobei X⁹ ausgewählt ist aus der Gruppe bestehend aus Wasserstoff, -OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen;
   wobei die Reste R⁷, R⁸ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   und wobei der Rest -NR⁷R⁸ auch ein Rest der chemischen Struktur **(x)** sein kann mit 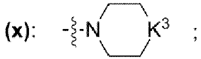 wobei K³ = -O-.
2.6 Verfahren nach einem oder mehreren der Punkte 2.1 bis 2.5, wobei Formaldehyd als Gas, als wässrige Lösung oder als Feststoff eingesetzt wird, bevorzugt als wässrige Lösung oder als Feststoff, noch bevorzugter als wässrigeLösung.
2.7 Verfahren nach einem oder mehreren der Punkte 2.1 bis 2.6, wobei man die mindestens eine Triacetonaminverbindung **(I)** mit Formaldehyd unter reduktiven Bedingungen in mindestens einem Lösungsmittel das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
2.8 Verfahren nach einem oder mehreren der Punkte 2.1 bis 2.7, wobei man die mindestens eine Triacetonaminverbindung **(I)** mit Formaldehyd unter reduktiven Bedingungen bei einer Temperatur im Bereich von 20 °C bis 350°C und einem Druck im Bereich von 2 bar bis 500 bar umsetzt.

Die vorliegende Erfindung betrifft in einem dritten Aspekt ein Verfahren gemäß den folgenden Punkten 3.1 bis 3.8.
3.1 Verfahren zur Herstellung einer *N*-methylsubstituierten Triacetonaminverbindung, dadurch gekennzeichnet, dass man mindestens eine Triacetonaminverbindung **(I)** mit Formaldehyd unter reduktiven Bedingungen umsetzt, wobei die Triacetonaminverbindung **(I)** die chemische Strukturen **(I-G)** mit 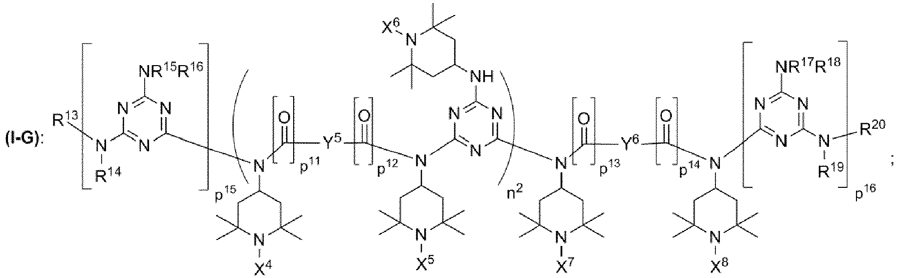
   wobei n² eine ganze Zahl aus dem Bereich 1 bis 20 ist;
   wobei p¹¹, p¹², p¹³, p¹⁴, p¹⁵, p¹⁶ unabhängig voneinander jeweils 0 oder 1 ist;
   wobei X⁴ = X⁵ = X⁶ = X⁷ = X⁸ = Wasserstoff;
   wobei Y⁵, Y⁶ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus (i), (ii) mit 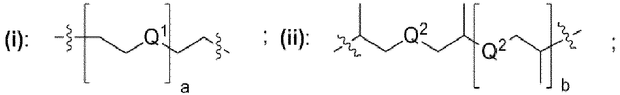 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
            wobei a eine ganze Zahl ausgewählt aus dem Bereich 1 bis 50 ist,
         wobei b eine ganze Zahl ausgewählt aus dem Bereich 0 bis 50 ist,
   und wobei Y⁵ auch eine direkte Bindung sein kann, falls mindestens einer von p¹¹ und p¹² den Wert 1 aufweist,
   und wobei Y⁶ auch eine direkte Bindung sein kann, falls mindestens einer von p¹³ und p¹⁴ den Wert 1 aufweist;
   wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus **(iii), (iv), (v)**, **(vi)**, **(vii)**, **(viii)**, **(ix)** mit 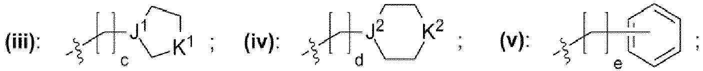 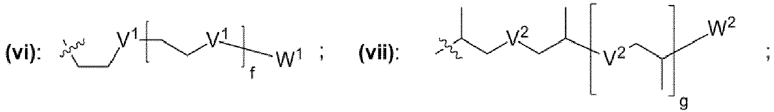 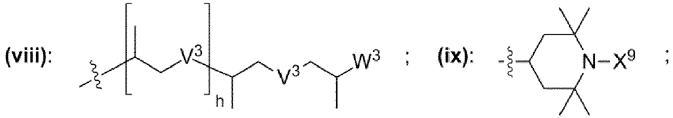 aufweist,
         wobei J¹, J² unabhängig voneinander jeweils ausgewählt aus der Gruppe bestehend aus CH, N sind,
         wobei K¹, K² unabhängig voneinander jeweils ausgewählt aus der Gruppe bestehend aus -O-, -NH-, -N(CH₃)-, -N(CH₂CH₃)-, -S-, -CH₂- sind,
         wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
         wobei W¹, W², W³ unabhängig voneinander jeweils ausgewählt aus der Gruppe bestehend aus H, Methyl, Ethyl sind,
         wobei c, d, e, f, g, h unabhängig voneinander jeweils eine ganze Zahl aus dem Bereich 0 bis 50 ist,
         wobei X⁹ = Wasserstoff,
      wobei in den chemischen Strukturen **(iii), (iv), (v), (vi), (vii), (viii), (ix)** mindestens ein an ein Kohlenstoffatom gebundener Wasserstoffrest durch einen Rest ausgewählt aus der Gruppe bestehend aus
      -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und wobei man reduktive Bedingungen dadurch einstellt, dass man die mindestens eine Triacetonaminverbindung **(I)** mit Formaldehyd in Gegenwart von Wasserstoff und in Gegenwart eines Trägerkatalysators umsetzt, wobei der Trägerkatalysator mindestens ein Metall **M** aufweist, wobei das Metall **M** ausgewählt ist aus der Gruppe bestehend aus Cr, Mo, Mn, Ni, Pd, Pt, Ru, Rh.
3.2 Verfahren nach Punkt 3.1, wobei p¹¹ = p¹² = p¹³ = p¹⁴ = 0 ist und wobei p¹⁵, p¹⁶ unabhängig voneinander jeweils 0 oder 1 ist.
3.3 Verfahren nach Punkt 3.1 oder 3.2, wobei
   Y⁵, Y⁶ unabhängig voneinander jeweils ausgewählt ist aus der Gruppe bestehend aus unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
3.4 Verfahren nach einem oder mehreren der Punkte 3.1 bis 3.3, wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(ix)** mit 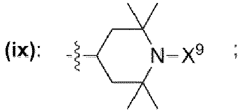 aufweist,
      wobei X⁹ = Wasserstoff.
3.5 Verfahren nach einem oder mehreren der Punkte 3.1 bis 3.4, wobei die Reste R¹³, R¹⁴, R¹⁵, R¹⁶, R¹⁷, R¹⁸, R¹⁹, R²⁰ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einem Rest, der eine chemischen Struktur **(ix)** mit 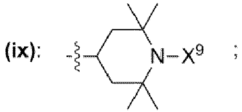 wobei X⁹ = Wasserstoff.
3.6 Verfahren nach einem oder mehreren der Punkte 3.1 bis 3.5, wobei Formaldehyd als Gas, als wässrige Lösung oder als Feststoff eingesetzt wird, bevorzugt als wässrige Lösung oder als Feststoff, noch bevorzugter als wässrige Lösung.
3.7 Verfahren nach einem oder mehreren der Punkte 3.1 bis 3.6, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
3.8 Verfahren nach einem oder mehreren der Punkte 3.1 bis 3.7, wobei man die mindestens eine Triacetonaminverbindung **(I)** mit Formaldehyd unter reduktiven Bedingungen bei einer Temperatur im Bereich von 20 °C bis 350°C und einem Druck im Bereich von 2 bar bis 500 bar umsetzt.

Die vorliegende Erfindung betrifft in einem vierten Aspekt ein Verfahren gemäß den folgenden Punkten 4.1 bis 4.7.
4.1 Verfahren zur Herstellung einer *N*-methylsubstituierten Triacetonaminverbindung, dadurch gekennzeichnet, dass man mindestens eine Triacetonaminverbindung **(I)** mit Formaldehyd unter reduktiven Bedingungen umsetzt,
   wobei die Triacetonaminverbindung **(I)** die chemische Strukturen **(I-H)** mit 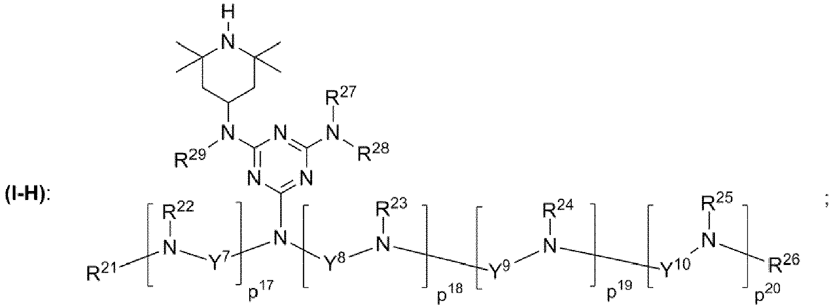
   wobei p¹⁷, p¹⁸, p¹⁹, p²⁰ unabhängig voneinander jeweils 0 oder 1 ist;
   wobei Y⁷, Y⁸, Y⁹, Y¹⁰ unabhängig voneinander jeweils ausgewählt ist aus der Gruppe bestehend aus
      unverzweigte oder verzweigte Alkylengruppe mit 1 bis 30 Kohlenstoffatomen,
      zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
      zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
      ein verbrückender Rest, der eine chemische Struktur ausgewählt aus der Gruppe bestehend aus (i), (ii) mit 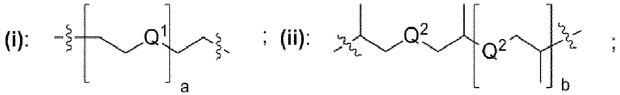 aufweist, wobei
         Q¹, Q² unabhängig voneinander jeweils ausgewählt aus der Gruppe bestehend aus -O-, -S-, -NH- oder -NR'- mit R' = unverzweigte oder verzweigte Alkylgruppe mit 1 bis 6 Kohlenstoffatomen sind,
         wobei a eine ganze Zahl ausgewählt aus dem Bereich 1 bis 50 ist,
         wobei b eine ganze Zahl ausgewählt aus dem Bereich 0 bis 50 ist,
   wobei die Reste R²¹, R²², R²³, R²⁴, R²⁵, R²⁶ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen,
      einer Gruppe mit der chemischen Struktur (xi) mit 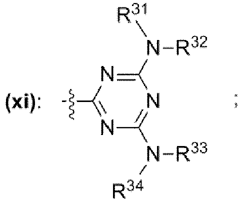 wobei die Reste R²⁷, R²⁸, R²⁹, R³¹, R³², R³³, R³⁴ unabhängig voneinander jeweils ausgewählt sind aus der Gruppe bestehend aus
      Wasserstoff,
      unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
      einem Rest, der eine chemischen Struktur ausgewählt aus der Gruppe bestehend aus (xii), (xiii), (xiv), **(xv), (xvi), (xvii), (xviii)** mit 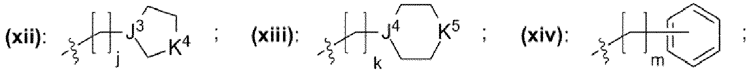 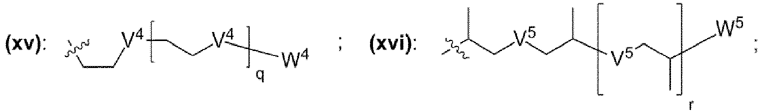 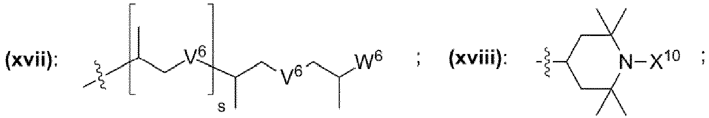 aufweist,
         wobei J³, J⁴ unabhängig voneinander jeweils ausgewählt aus der Gruppe bestehend aus CH, N sind,
         wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind,
         wobei V⁴, V⁵, V⁶ unabhängig voneinander jeweils ausgewählt aus der Gruppe bestehend aus -O-, -S-, -NH-, -NR"'- mit R'" = unverzweigte oder verzweigte Alkylgruppe mit 1 bis 6 Kohlenstoffatomen sind,
         wobei W⁴, W⁵, W⁶ unabhängig voneinander jeweils ausgewählt aus der Gruppe bestehend aus H, Methyl, Ethyl sind,
         wobei j, k, m, q, r, s unabhängig voneinander jeweils eine ganze Zahl aus dem Bereich 0 bis 50 ist, wobei X¹⁰ = Wasserstoff,
         wobei in den chemischen Strukturen **(xii), (xiii), (xiv), (xv), (xvi), (xvii), (xviii)** mindestens ein an ein Kohlenstoffatom gebundener Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur (xix) mit 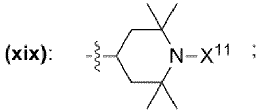 sein können, wobei X¹¹ = Wasserstoff,
   und wobei man reduktive Bedingungen dadurch einstellt, dass man die mindestens eine Triacetonaminverbindung **(I)** mit Formaldehyd in Gegenwart von Wasserstoff und in Gegenwart eines Trägerkatalysators umsetzt, wobei der Trägerkatalysator mindestens ein Metall **M** aufweist, wobei das Metall **M** ausgewählt ist aus der Gruppe bestehend aus Cr, Mo, Mn, Ni, Pd, Pt, Ru, Rh.
4.2 Verfahren nach Punkt 4.1, wobei
   Y⁷, Y⁸, Y⁹, Y¹⁰ unabhängig voneinander jeweils ausgewählt ist aus der Gruppe bestehend aus
   unverzweigte oder verzweigte Alkylengruppe mit 1 bis 30 Kohlenstoffatomen,
   zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.
4.3 Verfahren nach einem oder mehreren der Punkte 4.1 bis 4.2, wobei die Reste R²¹, R²², R²³, R²⁴, R²⁵, R²⁶ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen,
   einer Gruppe mit der chemischen Struktur (xi) mit 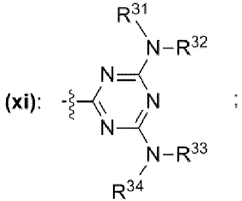 wobei die Reste R²⁷, R²⁸, R²⁹, R³¹, R³², R³³, R³⁴ unabhängig voneinander jeweils ausgewählt sind aus der Gruppe bestehend aus
   Wasserstoff,
   unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
   einem Rest, der eine chemischen Struktur **(xviii)** mit 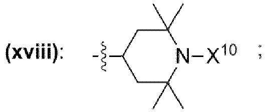 aufweist,
      wobei X¹⁰ = Wasserstoff,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 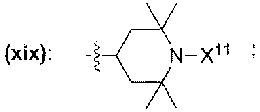 sein können, wobei X¹¹ = Wasserstoff.
4.4 Verfahren nach einem oder mehreren der Punkte 4.1 bis 4.3, wobei die Reste R²¹, R²², R²³, R²⁴, R²⁵, R²⁶ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einer Gruppe mit der chemischen Struktur **(xi)** mit 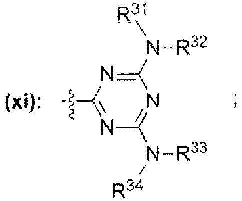 wobei die Reste R²⁷, R²⁸, R²⁹, R³¹, R³², R³³, R³⁴ unabhängig voneinander jeweils ausgewählt sind aus der Gruppe bestehend aus
   Wasserstoff,
   unverzweigte oder verzweigte Alkylgruppe mit 1 bis 12, bevorzugt 1 bis 6, Kohlenstoffatomen,
   einem Rest, der eine chemischen Struktur **(xviii)** mit 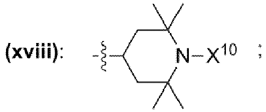 aufweist,
      wobei X¹⁰ = Wasserstoff,
   und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 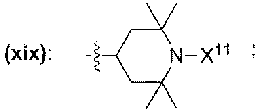 sein können, wobei X¹¹ ausgewählt ist aus der Gruppe bestehend aus Wasserstoff, OH, -O·, unverzweigte oder verzweigte Alkylgruppe mit 1 bis 10 Kohlenstoffatomen, unverzweigte oder verzweigte Alkoxygruppe mit 1 bis 10 Kohlenstoffatomen.
4.5 Verfahren nach einem oder mehreren der Punkte 4.1 bis 4.4, wobei Formaldehyd als Gas, als wässrige Lösung oder als Feststoff eingesetzt wird, bevorzugt als wässrige Lösung oder als Feststoff, noch bevorzugter als wässrige Lösung.
4.6 Verfahren nach einem oder mehreren der Punkte 4.1 bis 4.5, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.
4.7 Verfahren nach einem oder mehreren der Punkte 4.1 bis 4.6, wobei man die mindestens eine Triacetonaminverbindung **(I)** mit Formaldehyd unter reduktiven Bedingungen bei einer Temperatur im Bereich von 20 °C bis 350°C und einem Druck im Bereich von 2 bar bis 500 bar umsetzt.

### Allgemeine Begriffe

Im Sinne der Erfindung ist eine "unverzweigte oder verzweigte Alkylgruppe" ein einwertiger gesättigter Kohlenwasserstoffrest der allgemeinen chemische Struktur **(a)** mit 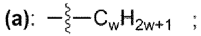 aufweist.

Die Kette an Kohlenstoffatomen "-C_{w}H_{2w+1}" kann dabei linear sein, dann handelt es sich um eine unverzweigte Alkylgruppe. Sie kann aber auch Verzweigungen aufweisen, dann handelt es sich um eine verzweigte Alkylgruppe.

Dabei ist w in der chemischen Struktur **(a)** eine ganze Zahl. w ist bei einer unverzweigten oder verzweigten Alkylgruppe mit 1 bis 30 Kohlenstoffatomen ausgewählt aus dem Bereich 1 bis 30. w ist bei einer unverzweigten oder verzweigten Alkylgruppe mit 1 bis 29 Kohlenstoffatomen ausgewählt aus dem Bereich 1 bis 29. w ist bei einer unverzweigten oder verzweigten Alkylgruppe mit 1 bis 12 Kohlenstoffatomen ausgewählt aus dem Bereich 1 bis 12. w ist bei einer unverzweigten oder verzweigten Alkylgruppe mit 1 bis 10 Kohlenstoffatomen ausgewählt aus dem Bereich 1 bis 10. w ist bei einer unverzweigten oder verzweigten Alkylgruppe mit 1 bis 8 Kohlenstoffatomen ausgewählt aus dem Bereich 1 bis 8. w ist bei einer unverzweigten oder verzweigten Alkylgruppe mit 1 bis 6 Kohlenstoffatomen ausgewählt aus dem Bereich 1 bis 6.

Im Sinne der Erfindung ist eine "unverzweigte oder verzweigte Alkylgruppe mit 1 bis 30 Kohlenstoffatomen" insbesondere ausgewählt aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl, *n*-Tricontyl.

Im Sinne der Erfindung ist eine "unverzweigte oder verzweigte Alkylgruppe mit 1 bis 12 Kohlenstoffatomen" insbesondere ausgewählt aus derGruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl.

Im Sinne der Erfindung ist eine "unverzweigte oder verzweigte Alkylgruppe mit 1 bis 10 Kohlenstoffatomen" insbesondere ausgewählt aus derGruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl.

Im Sinne der Erfindung ist eine "unverzweigte oder verzweigte Alkylgruppe mit 1 bis 8 Kohlenstoffatomen" insbesondere ausgewählt aus derGruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl. Im Sinne der Erfindung ist eine "unverzweigte oder verzweigte Alkylgruppe mit 1 bis 6 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl.

Der Begriff "unverzweigte oder verzweigte Alkylengruppe" bezeichnet im Sinne der Erfindung einen zweiwertigen gesättigten Kohlenwasserstoffrest, welcher durch die allgemeine chemische Struktur **(b)** mit 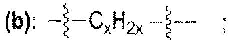 beschrieben werden kann. Die Kette an Koh!enstoffatomen"-CₓH₂ₓ" kann dabei linear sein, dann handelt es sich um eine unverzweigte Alkylengruppe. Sie kann aber auch Verzweigungen aufweisen, dann handelt es sich um eine verzweigte Alkylengruppe. Dabei ist x in der chemischen Struktur **(b)** eine ganze Zahl.

x ist bei einer unverzweigten oder verzweigten Alkylengruppe mit 1 bis 30 Kohlenstoffatomen ausgewählt aus dem Bereich 1 bis 30.

x ist bei einer unverzweigten oder verzweigten Alkylengruppe mit 1 bis 12 Kohlenstoffatomen ausgewählt aus dem Bereich 1 bis 12.

x ist bei einer unverzweigten oder verzweigten Alkylengruppe mit 1 bis 6 Kohlenstoffatomen ausgewählt aus dem Bereich 1 bis 6.

Im Sinne der Erfindung ist eine "zweiwertige gesättigte Kohlenwasserstoffgruppe mit 3 bis 30 Kohlenstoffatomen, welche mindestens einen gesättigten Ring aus 3 bis 30 Kohlenstoffatomen aufweist" insbesondere eine chemische Struktur **(c)** mit 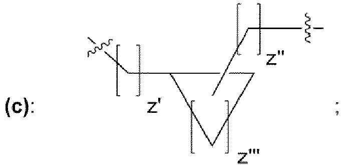 wobei z' eine ganze Zahl zwischen 0 und 27 ist; wobei z" eine ganze Zahl zwischen 0 und 27 ist; wobei z'" eine ganze Zahl zwischen 1 und 28 ist; und wobei gleichzeitig gilt, dass z' + z" + z'" ≤ 28.

Insbesondere ist eine "zweiwertige gesättigte Kohlenwasserstoffgruppe mit 3 bis 30 Kohlenstoffatomen, welche mindestens einen gesättigten Ring aus 3 bis 30 Kohlenstoffatomen aufweist" eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist", noch bevorzugter ""zweiwertige gesättigte Kohlenwasserstoffgruppe mit 3 bis 6 Kohlenstoffatomen, welche mindestens einen gesättigten Ring aus 3 bis 6 Kohlenstoffatomen aufweist".

Eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" weist im Sinne der Erfindung eine chemische Struktur **(c)** auf, wobei z' eine ganze Zahl zwischen 0 und 9 ist; wobei z" eine ganze Zahl zwischen 0 und 9 ist; wobei z'" eine ganze Zahl zwischen 1 und 10 ist; und wobei gleichzeitig gilt, dass z' + z" + z'" ≤ 10.

Bevorzugt ist eine "zweiwertige gesättigte Kohlenwasserstoffgruppe mit 3 bis 12 Kohlenstoffatomen, welche mindestens einen gesättigten Ring aus 3 bis 12 Kohlenstoffatomen aufweist" ausgewählt aus der Gruppe bestehend aus Cyclopropylen, Cyclobutylen, Cyclopentylen, Cyclohexylen, Cycloheptylen, Cyclooctylen, Cyclononylen, Cyclodecylen, Cycloundecylen, Cyclododecylen.

Eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" weist im Sinne der Erfindung eine chemische Struktur **(c)** auf, wobei z' eine ganze Zahl zwischen 0 und 3 ist; wobei z" eine ganze Zahl zwischen 0 und 3 ist; wobei z'" eine ganze Zahl zwischen 1 und 4 ist; und wobei gleichzeitig gilt, dass z' + z" + z'" ≤ 4.

Bevorzugt ist eine "zweiwertige gesättigte Kohlenwasserstoffgruppe mit 3 bis 6 Kohlenstoffatomen, welche mindestens einen gesättigten Ring aus 3 bis 6 Kohlenstoffatomen aufweist" ausgewählt aus der Gruppe bestehend aus Cyclopropylen, Cyclobutylen, Cyclopentylen, Cyclohexylen.

Im Sinne der Erfindung ist eine "zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind" insbesondere eine "zweiwertige Kohlenwasserstoffgruppe mit 6 bis 30 Kohlenstoffatomen, von denen 6, 10 oder 14 Kohlenstoffatome in einem aromatischen System vorliegen und die übrigen Kohlenstoffatome, falls vorhanden, gesättigt sind" und bevorzugter ausgewählt aus der Gruppe bestehend aus Naphthylen, Anthrylen, Phenanthrylen sowie der folgenden chemischen Struktur **(d):** 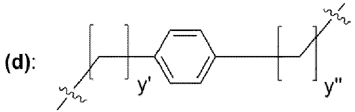 wobei y' eine ganze Zahl zwischen 0 und 24 ist; wobei y" eine ganze Zahl zwischen 0 und 24 ist; und wobei gleichzeitig gilt, dass y' + y" ≤ 24.

Noch bevorzugter handelt es sich dabei um eine "zweiwertige Kohlenwasserstoffgruppe mit 6 bis 30 Kohlenstoffatomen, von denen 6 oder 10 Kohlenstoffatome in einem aromatischen System vorliegen und die übrigen Kohlenstoffatome, falls vorhanden, gesättigt sind" und am bevozugtesten ist diese dann ausgewählt aus der Gruppe bestehend aus Naphthylen, sowie der folgenden chemischen Struktur **(d):** 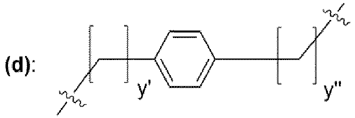 wobei y' eine ganze Zahl zwischen 0 und 24 ist; wobei y" eine ganze Zahl zwischen 0 und 24 ist; und wobei gleichzeitig gilt, dass y' + y" ≤ 24.

Im Sinne der Erfindung ist eine "unverzweigte oder verzweigte Alkoxygruppe" ein organischer Rest der chemischen Struktur 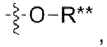 in welchem R** eine unverzweigte oder verzweigte Alkylgruppe ist. Bei einer "unverzweigten oder verzweigten Alkoxygruppe mit 1 bis 30 Kohlenstoffatomen" ist R** eine unverzweigte oder verzweigte Alkylgruppe mit 1 bis 30 Kohlenstoffatomen.

Bei einer "unverzweigten oder verzweigten Alkoxygruppe mit 1 bis 10 Kohlenstoffatomen" ist R** eine unverzweigte oder verzweigte Alkylgruppe mit 1 bis 10 Kohlenstoffatomen.

Im Sinne der Erfindung ist eine "unverzweigte oder verzweigte Alkoxygruppe mit 1 bis 10 Kohlenstoffatomen" insbesondere ausgewählt aus der Gruppe bestehend aus Methoxy, Ethoxy, *n*-Propoxy, *iso*-Propoxy, *n*-Butoxy, *sec*-Butoxy, *iso*-Butoxy, *ter*t-Butoxy, *n*-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, *n*-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, *n*-Heptoxy, *n*-Octoxy, *n*-Nonoxy, *n*-Decoxy.

Im Sinne der Erfindung ist eine "unverzweigte oder verzweigte Acylgruppe mit 1 bis 30 Kohlenstoffatomen" ein organischer Rest der chemischen Struktur 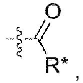 in welchem R* ein unverzweigter oder verzweigter Alkylrest mit 1 bis 29 Kohlenstoffatomen ist.

Insbesondere ist R* ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl.

"-O·" bezeichnet im Sinne der Erfindung einen radikalischen Sauerstoffrest.

Im Sinne der Erfindung bedeutet die Formulierung "mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -NH(CH₂CH₃), -N(CH₃)(CH₂CH₃) ersetzt sein kann", dass die betreffende Gruppe unsubstituiert vorliegt oder in der betreffenden Gruppe mindestens ein an ein Kohlenstoffatom gebundener Wasserstoffrest, bevorzugt 1 bis 5, noch bevorzugter 1 bis 3, am bevorzugtesten 1 bis 2 an das selbe oder unterschiedliche Kohlenstoffatom(e) gebundene(r) Wasserstoffrest(e), durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) substituiert ist.

### Erfindungsgemäßes Verfahren

Formaldehyd wird insbesondere im erfindungsgemäßen Verfahren als Gas, als wässrige Lösung oder als Feststoff eingesetzt werden. Bevorzugt wird Formaldehyd im erfindungsgemäßen Verfahren als wässrige Lösung oder als Feststoff (z. B. als Paraformaldehyd) eingesetzt.

In der noch bevorzugteren Ausführungsform, in der Formaldehyd als wässrige Lösung eingesetzt wird, beträgt die Konzentration des Formaldehyds in der Lösung 1.0 bis 37 Gew.-% (w/w, "%" bezieht sich auf das Gewicht des Formaldehyds bezogen auf das Gesamtgewicht der wässrigen Lösung). Bei 37 Gew.-% an Formaldehyd enthalten zum Beispiel 100 g wässrige Lösung 37 g Formaldehyd.

Das erfindungsgemäße Verfahren wird unter reduktiven Bedingungen durchgeführt. Unter "reduktiven Bedingungen" sind die Bedingungen zu verstehen, bei welchen das im Reaktionsschema **<1>** gezeigte Imin durch Wasserstoffanlagerung in das entsprechende Amin überführt wird.

Man stellt im erfindungsgemäßen Verfahren reduktive Bedingungen dadurch ein, dass man die mindestens eine Triacetondiaminverbindung **(I)** mit der mindestens einen Carbonylverbindung **(II)** in Gegenwart von Wasserstoff und in Gegenwart eines Trägerkatalysators umsetzt, wobei der Trägerkatalysator mindestens ein Metall **M** aufweist, wobei das Metall **M** ausgewählt ist aus der Gruppe bestehend aus Cr, Mo, Mn, Ni, Pd, Pt, Ru, Rh; noch bevorzugter ausgewählt ist aus der Gruppe bestehend aus Cr, Ni, Pd, Pt; am bevorzugtesten ausgewählt ist aus der Gruppe bestehend aus Ni, Pd.

Die Verwendung eines Trägerkatalysators aufweisend mindestens ein Metall **M** ist wesentlich für das erfindungsgemäße Verfahren: solche Trägerkatalysatoren sind dem Fachmann bekannt und insbesondere in der EP 0 302 020 A2 beschrieben.

"Trägerkatalysator, wobei dieser mindestens ein Metall **M** aufweist" bedeutet insbesondere, dass das Metall **M,** welches bevorzugt im elementaren Zustand vorliegt, auf einem dem Fachmann bekannten Träger aufgebracht ist, welcher beispielsweise insbesondere ausgewählt aus der Gruppe bestehend aus Aktivkohle, Calciumcarbonat, Aluminiumoxid, Titandioxid, insbesondere aus der Gruppe bestehend aus Aluminiumoxid, Aktivkohle sein kann.

Der Anteil an Metall **M** im Trägerkatalysator ist dabei nicht besonders beschränkt und liegt insbesondere im Bereich 0.1 bis 30 Gew.-%, bevorzugt 1 bis 10 Gew.-%, bevorzugter 5 Gew.-%. Dabei bedeutet Gew.-% das Gesamtgewicht aller vom jeweiligen Trägerkatalysator umfassten Metalle **M** bezogen auf das Gesamtgewicht des vom jeweiligen Trägerkatalysator umfassten Trägers.

Ohne einen solchen Trägerkatalysator würde man nur unerwünschte Produkte erhalten. C. Harries beschreibt zum Beispiel auf Seiten 220 bis 222 seines Artikels "Untersuchungen über die cyclischen Acetonbasen" in Justus Liebigs Annalen der Chemie, Band 417, 1918, Seiten 107 bis 191 eine Umsetzung von 4-Amino-2,2,6,6-Tetramethylaminopiperidin mit Acetanhydrid ohne Trägerkatalysator, was zu hohen Ausbeuten an der entsprechenden, hier unerwünschten Amidverbindung führt.

Das erfindungsgemäße Verfahren kann man lösungsmittelfrei oder auch in mindestens einem Lösungsmittel durchführen, bevorzugt in mindestens einem Lösungsmittel. Als Lösungsmittel eignen sich dabei alle Lösungsmittel, in denen sich die Reaktanden gut lösen und welche auch keinen störenden Einfluss auf das erfindungsgemäße Verfahren haben. Insbesondere ist das Lösungsmittel ausgewählt aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser; bevorzugt ist das Lösungsmittel ausgewählt aus der Gruppe bestehend aus aliphatische Lösungsmittel, aromatische Lösungsmittel, Ether, Alkohole, Wasser; bevorzugter ist das Lösungsmittel ausgewählt aus der Gruppe bestehend aus aliphatische Lösungsmittel, aromatische Lösungsmittel, Ether, Alkohole, Wasser; noch bevorzugter ist das Lösungsmittel ausgewählt aus der Gruppe bestehend aus aliphatische Lösungsmittel, aromatische Lösungsmittel, Alkohole, Wasser. Das Lösungsmittel ist besonders bevorzugt ausgewählt aus aromatischen Lösungsmittel (insbesondere Toluol), Alkohole (insbesondere Methanol).

Aliphatische Lösungsmittel sind insbesondere ausgewählt aus der Gruppe bestehend aus Pentan, Hexan, Heptan, Octan, Decan, Cyclopentan, Cyclohexan, Methylcyclohexan, Petrolether.

Aromatische Lösungsmittel sind insbesondere ausgewählt aus der Gruppe bestehend aus Benzol, Toluol, Xylol, Ethylbenzol, Cumol, Brombenzol, Chlorbenzol, Dichlorbenzol, Furan, bevorzugt Toluol.

Ether sind insbesondere ausgewählt aus der Gruppe bestehend aus Diethylether, Dipropylether, Dibutylether, Methyl-*tert-*Butylether, Ethylenglykolmonomethylether, Ethylenglykolmonoethylether, Ethylenglykoldimethylether, Ethylenglykoldiethylether, Diethylenglykolmonomethylether, Diethylenglykolmonoethylether, Diethylenglykoldimethylether, Diethylenglykoldiethylether, Triethylenglykolmonomethylether, Triethylenglykolmonoethylether, Triethylenglykoldimethylether, Triethylenglykoldiethylether, Polyethylenglykolmonomethylether, Polyethylenglykolmonoethylether, Polyethylenglykoldimethylether, Polyethylenglykoldiethylether, 1,4-Dioxan, 1,3-Dioxan, Tetrahydrofuran.

Halogenierte Lösungsmittel sind insbesondere ausgewählt aus der Gruppe bestehend aus Dichlormethan, Chloroform, Tetrachlormethan.

Amide sind insbesondere ausgewählt aus der Gruppe bestehend aus Dimethylformamid, Dimethylacetamid.

Thioverbindungen sind insbesondere ausgewählt aus der Gruppe bestehend aus Dimethylsulfoxid, Sulfolan.

Carbonsäuren sind insbesondere ausgewählt aus der Gruppe bestehend aus Ameisensäure, Essigsäure, Propionsäure, Butansäure, Pentansäure.

Alkohole, sind insbesondere ausgewählt aus der Gruppe bestehend aus
Methanol, Ethanol, Propanol, *iso*-Propanol, 1,2-Propandiol, 1,3-Propandiol, Glycerin, Butanol, *sec*-Butanol, *iso*-Butanol, *tert*-Butanol, 1,2-Butandiol, 1,3-Butandiol, 1,4-Butandiol, 1-Pentanol, 2-Pentanol, 3-Pentanol, *tert*-Amylalkohol, 1,2-Pentandiol, 1,3-Pentandiol, 1,4-Pentandiol, 1,5-Pentandiol, Cyclopentanol, Hexanol, Cyclohexanol Heptanol, Octanol, Nonanol, Decanol, Ethylenglykol, Diethylenglykol, Triethylenglykol, Polyethylenglykol, Benzylalkohol, Phenol; bevorzugt ausgewählt aus Methanol, Ethanol, *n*-Propanol, *iso*-Propanol; bevorzugt Methanol.

Das erfindungsgemäße Verfahren lässt sich kontinuierlich oder nicht kontinuierlich, also batchweise, durchführen.

Die Reaktionszeit hängt vom Fortgang des Verfahrens und vom gewünschten Umsatz ab - üblicherweise strebt man einen größtmöglichen Umsatz an und führt das erfindungsgemäße Verfahren so lange fort, bis kein Eduktumsatz mehr beobachtet werden kann.

Die Temperatur beim erfindungsgemäßen Verfahren ist nicht beschränkt und liegt bevorzugt im Bereich von 20 °C bis 350 °C, bevorzugter im Bereich von 50 °C bis 300 °C, noch bevorzugter im Bereich von 50 °C bis 250 °C, am bevorzugtesten im Bereich von 70 °C bis 200 °C, noch bevorzugter bei 80 °C bis 140 °C.

Der Druck beim erfindungsgemäßen Verfahren ist nicht beschränkt und liegt bevorzugt im Bereich von 2 bar bis 500 bar, bevorzugter im Bereich von 5 bar bis 350 bar, noch bevorzugter im Bereich von 15 bar bis 300 bar, noch bevorzugter 20 bis 42 bar.

Die obigen Temperaturbereiche und Druckbereiche können natürlich auch in Kombination vorliegen. So kann man das Verfahren bevorzugt bei einer Temperatur im Bereich von 20 °C bis 350 °C, [bevorzugter im Bereich von 50 °C bis 300 °C, noch bevorzugter im Bereich von 50 °C bis 250 °C, am bevorzugtesten im Bereich von 70 °C bis 200 °C, noch bevorzugter bei 80 °C - 140 °C] und einem Druck im Bereich von 2 bar bis 500 bar [bevorzugt im Bereich von 2 bar bis 500 bar, bevorzugter im Bereich von 5 bar bis 350 bar, noch bevorzugter im Bereich von 15 bar bis 300 bar, noch bevorzugter 20 bis 42 bar] durchführen.

Dieses Verfahren löst die erfindungsgemäßen Aufgaben. Besonders vorteilhaft ist, dass bei der Reaktion lediglich Wasser als Nebenprodukt entsteht. Zudem kann der geringe Überschuss an Formaldehyd entweder durch Hydrierung in Methanol überführt werden oder destillativ abgetrennt und gegebenenfalls zurückgeführt werden.
Die Aufarbeitung des Rohproduktes ist daher sehr einfach: Der Katalysator wird durch Filtration entfernt (vorstellbar wäre technisch auch die Verwendung eines Festbettkatalysators, so dass auch dieser Schritt entfallen würde), anschließend wird das Rohprodukt destillativ aufgereinigt. Es fallen bei der Destillation lediglich Methanol (kann recyclelt werden), Wasser, das Produkt und gegebenenfalls Formaldehyd (kann ebenfalls recyclelt werden) an.

Im Gegensatz zu den Verfahren nach dem Stand der Technik muss also keine Abtrennung von den gebildeten Salzen erfolgen (erfolgt im Allgemeinen durch Extraktion mit einem zusätzlichen Lösungsmittel), zudem fallen die genannten Salze (bzw. deren wässrige Lösung) nicht als Abfallstrom an.

Zusätzlich vorteilhaft ist bei dem erfindungsgemäßen Verfahren, dass zusätzlich zur Einführung der Methylgruppe am Piperidinstickstoff (*N¹*) auch andere Aminogruppen simultan methyliert werden können. In diesem Fall werden also zwei oder mehr Methylgruppen simultan eingeführt.

Ein weiterer Vorteil besteht darin, dass auch die Anwesenheit von tertiären Aminogruppen toleriert wird, ohne dass diese in die quartären Ammoniumsalze überführt werden.

Die nachfolgenden Beispiele sollen die Erfindung näher erläutern, ohne dass die Erfindung auf diese Ausführungsformen beschränkt sein soll.

### Beispiele

Paraformaldehyd wurde erworben von Sigma-Aldrich (Reinheit 95 %).

Die verwendete 37 Gew.-%-ige Formalinlösung wurde erworben von der Firma Sigma Aldrich.

Die in den nicht erfindungsgemäßen Beispielen **E1** bis **E12** genutzten Edukte waren die von der Firma Evonik Industries AG vertriebenen.

Das im erfindungsgemäßen Beispiel **E13** verwendete Edukt wurde von der Firma Cytec erhalten (Produktname: Cyasorb 3346).

Das im nicht erfindungsgemäßen Beispiel **E14** verwendete Edukt wurde von derFirma Beijing Huashan Auxiliary erworben (Produktname: Tinuvin 770).

Der verwendete Pd/ Aktivkohle - Katalysator war der von der Evonik Industries AG vertriebene (Produktname: E 196 NN/W 10 %).

### Beispiele E1 - E10

In einen 2 L - Druckautoklav wurden 1.5 mol der jeweiligen Triacetonaminverbindung ("Edukt" gemäß Tabelle 1), 3 mmol Palladium (als Pd/ Aktivkohle - Katalysator) und 400 ml Methanol gegeben. Im Anschluss wurde eine wässrige Lösung von Formaldehyd (37 Gew.-% an Formaldehyd, wobei die %-Angabe bezogen auf das Gesamtgewicht der Lösung zu verstehen ist, d.h. 100 g Lösung enthalten 37 g Formaldehyd) in der Menge gemäß Tabelle 1 hinzugefügt und der Reaktor verschlossen.

Unter Rühren wurde Wasserstoff aufgedrückt (40 bar H₂). Es wurde bei einer Temperatur von 80 bis 140 °C und einem Druck von 20 bis 42 barso lange hydriert, bis keine nennenswerte Wasserstoffaufnahme mehr zu beobachten war.

Der Reaktor wurde danach abgekühlt und entspannt. Das Rohprodukt wurde ausgetragen, filtriert und anschließend zunächst das Lösungsmittel entfernt (80 - 120 °C, Normaldruck). Dann wurde per Gaschromatographie (= GC, Agilent 5890 bzw. 7890, FID-Detektor) die Ausbeute an Produkt und gegebenenfalls die des/ der Nebenprodukte(s) (**E2**, **E3, E7, E9, E10**) im erhaltenen Rohprodukt bestimmt (siehe Tabelle 1, Strukturen in der Spalte "Rohprodukt"). Im Falle der Beispiele **E1** bis **E6** wurde zusätzlich der Rückstand anschließend mittels einer Vakuumdestillation gereinigt und die Ausbeute an Hauptprodukt per GC bestimmt.

Die Ergebnisse sind in der Tabelle 1 zusammengestellt.

### Beispiele E11 - E14

In einen 1 L-Druckautoklavwurde die in Tabelle 2 angegebene Menge der jeweiligen Triacetonaminverbindung ("Edukt" gemäß Tabelle 2), 0.2 mol.-% Pd (mol-% bezogen auf die Menge des Edukts) als Pd/ Aktivkohle - Katalysator, und 250 ml Lösungsmittel (Methanol oder Toluol) gegeben. Im Anschluss wurde Paraformaldeyhd in der Menge gemäß Tabelle 2 hinzugefügt, mit 50 ml Lösungsmittel nachgespült und der Reaktor verschlossen. Unter Rühren wurde Wasserstoff aufgedrückt (40 bar H₂). Es wurde bei einer Temperatur von 80 bis 140 °C und einem Druck von 20 bis 42 bar so lange hydriert, bis keine nennenswerte Wasserstoffaufnahme mehr zu beobachten war. Der Reaktor wurde danach abgekühlt und entspannt. Das Rohprodukt wurde ausgetragen, filtriert und anschließend zunächst das Lösungsmittel entfernt (80 - 120 °C, Normaldruck). Dann wurde im Falle der Beispiele **E11, E12, E14** per Gaschromatographie (= GC, Agilent 5890 bzw. 7890, FID-Detektor), im Falle des erfindungsgemäßen Beispiels **E13** mittels MS-ESI die Ausbeute an Produkt im erhaltenen Rohprodukt bestimmt (siehe Tabelle 2, Strukturen in der Spalte "Rohprodukt"). Im Falle des Beispiels **E11** wurde der Rückstand anschließend soweit möglich mittels einer Vakuumdestillation gereinigt.
Die Ergebnisse sind in der Tabelle 2 zusammengestellt.

**Tabelle 1**

| Beispiel Nr. | Ansatz | | Rohprodukt | | inprodukt | |
|---|---|---|---|---|---|---|
| | Struktur Edukt | wässrige FormalinLösung (37 Gew.-% Formaldehyd) | Struktur | GC-Analyse | GC-Analyse | Ausbeute |
| | (i) Molmasse Edukt | (i) eingesetzte Menge Formalin-Lsg. (37 Gew.-% Formaldehyd in Wasser) in g | | Flächen-% | Gehalt [Flächen - %] | Reinfraktion [%; bezogen auf eingesetzte Menge Edukt in mol] |
| | (ii) eingesetzte Menge Edukt in g [bzw. mol] | | | | | |
| | | (ii) eingesetzte Menge an Formaldehyd in mol | | | | |
| | | (iii) Moläquivalente [= "Moläq.] Formaldehyd, bezogen auf Edukt | | | | |
| **E1** | 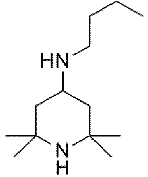 (i) 212.4 g/mol | (i) 267.8 g | 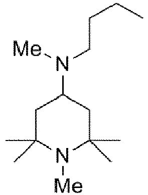 | 97.9 | 98.3 | 93.7 |
| | | (ii) 3.30 mol | | | | |
| | | (iii) 2.2 Moläq. | | | | |
| | (ii) 321.4 g [1.5 mol] | | | | | |
| **E2** | 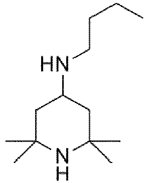 | (i) 133.9 g | 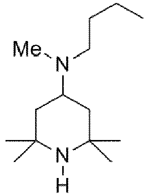 | 88.6 | 99.4 | 77.8 |
| | | (ii) 1.65 mol | | | | |
| | | (iii) 1.1 Moläq. | | | | |
| | (i) 212.4 g/mol | | 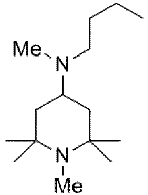 Nebenprodukt: | 10.4 | | |
| | (ii) 321.4 g [1.5 mol] | | | | | |
| E3 | 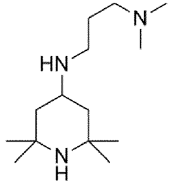 | (i) 267.8 g | 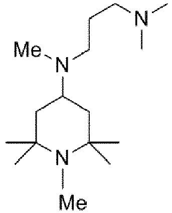 | 86.8 | 99.7 | 61.8 |
| | | (ii) 3.30 mol | | | | |
| | | (iii) 2.2 Moläq. | | | | |
| | (i) 241.4 g/mol | | 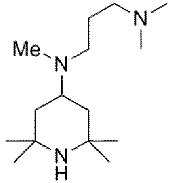 Nebenprodukt: | 12.5 | | |
| | (ii) 362.1 g [1.50 mol] | | | | | |
| **E4** | 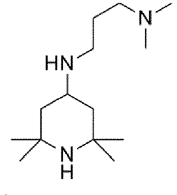 (i) 241.4 g/mol | (i) 121.7 g | 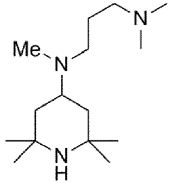 | 97.6 | 98.8 | 82.2 |
| | | (ii) 1.50 mol | | | | |
| | | (iii) 1.0 Moläq. | | | | |
| | (ii) 365.4 g [1.51 mol] | | | | | |
| **E5** | 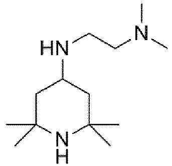 (i) 227.4 g/mol | (i) 316.5 g | 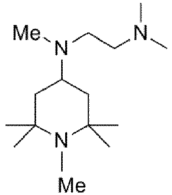 | 97.5 | 99.9 | 72.4 |
| | | (ii) 3.90 mol | | | | |
| | | (iii) 2.6 Moläq | | | | |
| | (ii) 341.8 g [1.50 mol] | | | | | |
| **E6** | 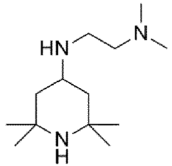 (i) 227.4 g/mol | (i) 63.3 g | 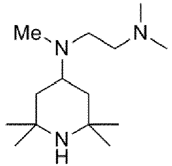 | 98.9 | 98.8 | 86.7 |
| | | (ii) 0.78 mol | | | | |
| | | (iii) 1.0 Moläq. | | | | |
| | (ii) 178.0 g [0.78 mol] | | | | | |
| **E7** | 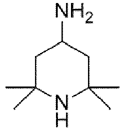 | (i) 401.8 g | 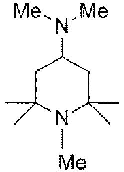 | 76.0 | 99.3 | 58.3 |
| | | (ii) 4.95 mol | | | | |
| | | (iii) 3.3 Moläq | | | | |
| | | | | | | |
| | (i) 156.3 g/mol | | | | | |
| | (ii) 238.0 g [1.5 mol] | | Neben produkt: | 21.5 | | |
| | | | 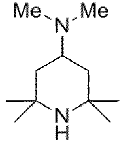 | | | |
| **E8** | 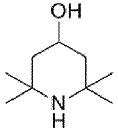 | (i) 89.3 g | 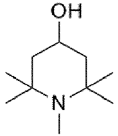 | 98.5 | - | - |
| | | (ii) 1.10 mol | | | | |
| | | (iii) 1.1 Moläq. | | | | |
| | (i) 157.3 g/mol | | | | | |
| | (ii) 157.0 g | | | | | |
| | (iii) 1.0 mol | | | | | |
| **E9** | 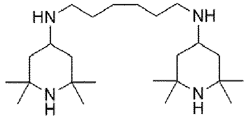 | (i) 89.2 g | 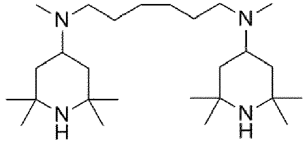 | 80.4 | - | - |
| | | (ii) 1.10 mol | | | | |
| | | (iii) 2.2 Moläq. | | | | |
| | (i) 394.7 g/mol | | | | | |
| | (ii) 198.3 g [0.5 mol] | | 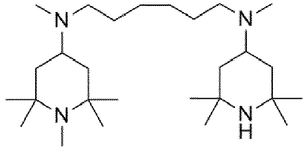 Nebenprodukte: | 18.0 | | |
| | | | 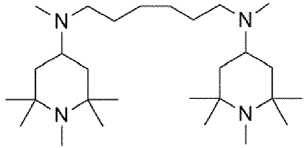 | 1.1 | | |
| **E10** | 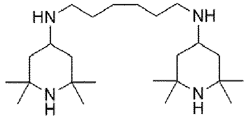 | (i) 170.4 g | 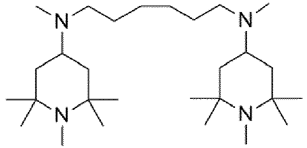 | 90.5 | - | - |
| | | (ii) 2.10 mol | | | | |
| | | (iii) 4.2 Moläq. | | | | |
| | (i) 394.7 g/mol | | Nebenprodukt: | 8.3 | | |
| | (ii) 198.3 g [0.5 mol] | | 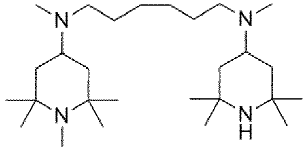 | | | |

**Tabelle 2**

| Beispiel Nr. | Ansatz | | | Rohprodukt | | Reinprodukt | |
|---|---|---|---|---|---|---|---|
| | Struktur Edukt | Paraformaldehyd (Reinheit 95 %) | Lösungsmittel | Struktur | GC-Analyse | GC-Analyse | Ausbeute |
| | (i) Molmasse Edukt | (i) eingesetzte Menge an Paraformaldehyd in g | | | Flächen-% | Gehalt [Flächen - %] | Reinfraktion [%; bezogen auf eingesetzte Menge Edukt in mol] |
| | (ii) eingesetzte Menge Edukt in g [bzw. mol] | | | | | | |
| | | (ii) eingesetzte Menge an Formaldehyd in mol (errechnet unter Berücksichtigung der Reinheit von 95 %) | | | | | |
| | | (iii) Moläquivalente [= "Moläq.] Formaldehyd, bezogen auf Edukt | | | | | |
| **E11** | 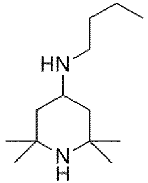 | (i) 52.2 g | Methanol | 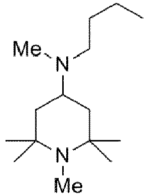 | 97.9 | 98.3 | 93.7 |
| | | (ii) 1.65 mol | | | | | |
| | | (iii) 2.2 Moläq. | | | | | |
| | (i) 212.4 g/mol | | | | | | |
| | (ii) 160.9 g | | | | | | |
| | (iii) 0.76 mol | | | | | | |
| **E12** | | (i) 52.2 g | Toluol | 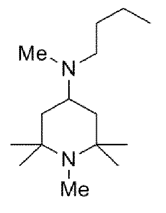 | 99.6 | | |
| | | (ii) 1.65 mol | | | | | |
| | 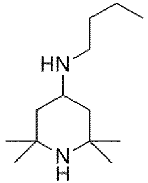 (i) 212.4 g/mol | (iii) 2.2 Moläq. | | | | | |
| | (ii) 160.9 g | | | | | | |
| | (iii) 0.76 mol | | | | | | |
| **E13** | 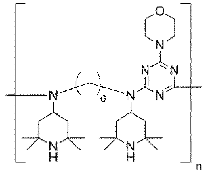 Polymer aus 1,6-*N*¹, *N*⁶-Bis (2,2,6,6-tetramethyl-4-piperidinyl)-hexandiamin mit2,4-dichloro-6-(4-morpholinyl)-1,3,5-triazin (CAS-Nr: 082451-48-7); Monomereinheit: (i) 558.8 g/ mol Monomereinheit | (i) 20.9 g | Toluol | 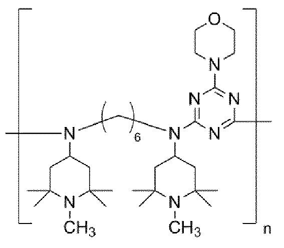 | MS-ESI zeigt vollständige Umsetzung | - | 95.0 |
| | | (ii) 0.66 mol | | | | | |
| | | (iii) 2.2 Moläq. pro Monomereinheit | | | | | |
| | (ii) 167.0 g [0.3 mol Monomereinheiten] | | | | | | |
| **E14** | 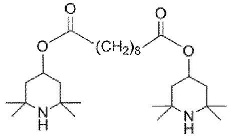 | (i) 34.8 g | MeOH | 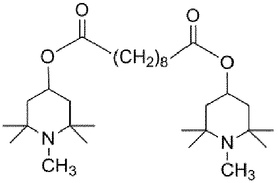 | 94.1 | - | - |
| | | (ii) 1.10 mol | | | | | |
| | | (iii) 2.2 Moläq. | | | | | |
| | (i) 480.7 g/mol | | | | | | |
| | (ii) 241.1 g [0.50 mol] | | | | | | |

## Patentansprüche

1. Verfahren zur Herstellung einer *N*-methylsubstituierten Triacetonaminverbindung,
**dadurch gekennzeichnet, dass** man mindestens eine Triacetonaminverbindung **(I)** mit Formaldehyd unter reduktiven Bedingungen umsetzt,
wobei die Triacetonaminverbindung **(I)** ausgewählt ist aus der Gruppe bestehend aus den chemischen Strukturen **(I-F), (I-G), (I-H)** mit 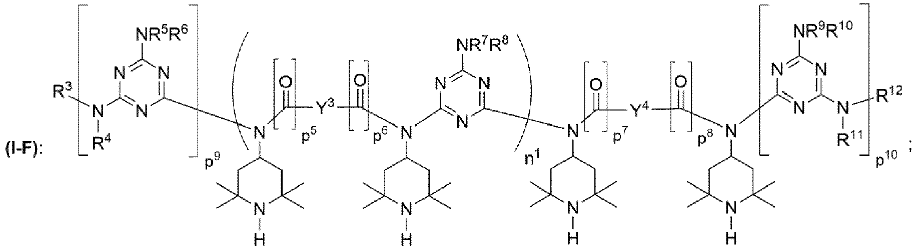 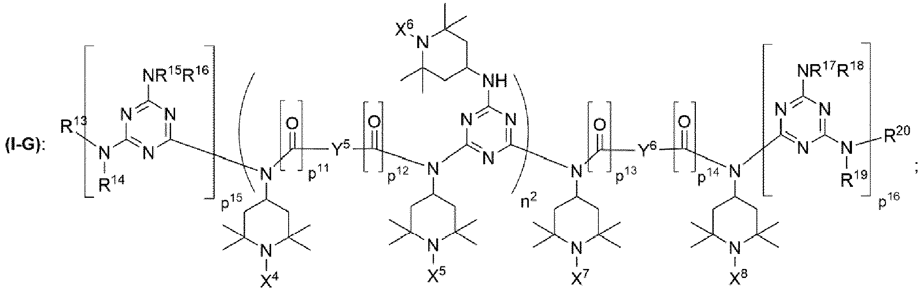 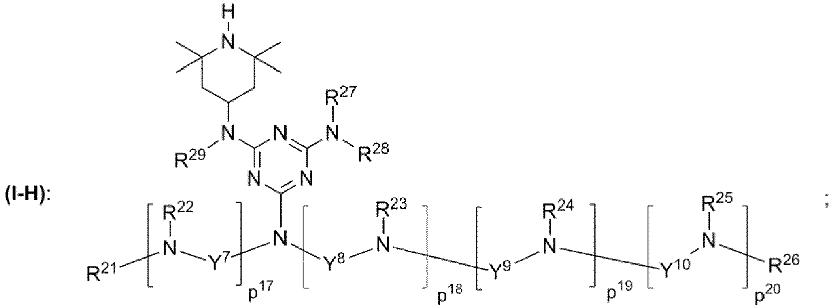
wobei n¹, n² unabhängig voneinander jeweils eine ganze Zahl aus dem Bereich 1 bis 20 ist;
wobei p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰ unabhängig voneinander jeweils 0 oder 1 ist;
wobei X⁴ = X⁵ = X⁶ = X⁷ = X⁸ = Wasserstoff;
wobei Y³, Y⁴, Y⁵, Y⁶, Y⁷, Y⁸, Y⁹, Y¹⁰ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist,
zweiwertige Kohlenwasserstoffgruppe mit 6 bis 30 Kohlenstoffatomen, von denen mindestens 6 Kohlenstoffatome in einem aromatischen System vorliegen und die übrigen Kohlenstoffatome, falls vorhanden, gesättigt sind,
ein verbrückender Rest, der eine chemische Struktur ausgewählt aus der Gruppe bestehend aus (i), (ii) mit 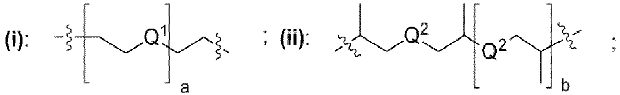
aufweist, wobei
Q¹, Q² unabhängig voneinander jeweils ausgewählt aus der Gruppe bestehend aus -O-, -S-, -NH- oder ―NR'- mit R' = unverzweigte oder verzweigte Alkylgruppe mit 1 bis 6 Kohlenstoffatomen sind,
wobei a eine ganze Zahl ausgewählt aus dem Bereich 1 bis 50 ist,
wobei b eine ganze Zahl ausgewählt aus dem Bereich 0 bis 50 ist,
und wobei Y³ auch eine direkte Bindung sein kann, falls mindestens einer von p⁵ und p⁶ den Wert 1 aufweist,
und wobei Y⁴ auch eine direkte Bindung sein kann, falls mindestens einer von p⁷ und p⁸ den Wert 1 aufweist,
und wobei Y⁵ auch eine direkte Bindung sein kann, falls mindestens einer von p¹¹ und p¹² den Wert 1 aufweist,
und wobei Y⁶ auch eine direkte Bindung sein kann, falls mindestens einer von p¹³ und p¹⁴ den Wert 1 aufweist;
wobei die Reste R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰ unabhängig voneinander jeweils ausgewählt sind aus der Gruppe bestehend aus Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(C H₃)(CH₂CH₃) ersetzt sein kann,
einem Rest, der eine chemischen Struktur ausgewählt aus der Gruppe bestehend aus (iii), (iv), (v), (vi), (vii), (viii), (ix) mit 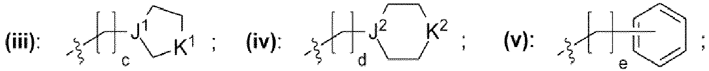 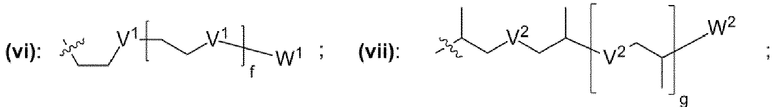 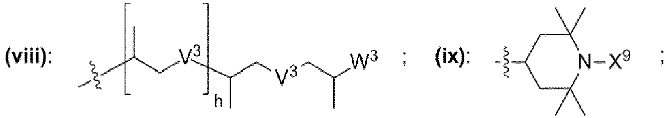 aufweist,
wobei J¹, J² unabhängig voneinander jeweils ausgewählt aus der Gruppe bestehend aus CH, N sind,
wobei K¹, K² unabhängig voneinander jeweils ausgewählt aus der Gruppe bestehend aus -O-, -NH-, -N(CH₃)-, -N(CH₂CH₃)-, -S-, -CH₂- sind,
wobei V¹, V², V³ unabhängig voneinander jeweils ausgewählt aus der Gruppe bestehend aus -O-, -S-, -NH-, -NR"- mit R" = unverzweigte oder verzweigte Alkylgruppe mit 1 bis 6 Kohlenstoffatomen sind,
wobei W¹, W², W³ unabhängig voneinander jeweils ausgewählt aus der Gruppe bestehend aus H, Methyl, Ethyl sind,
wobei c, d, e, f, g, h unabhängig voneinander jeweils eine ganze Zahl aus dem Bereich 0 bis 50 ist,
wobei X⁹ = Wasserstoff,
wobei in den chemischen Strukturen (iii), (iv), (v), (vi), (vii), (viii), (ix) mindestens ein an ein Kohlenstoffatom gebundener Wasserstoffrest durch einen Rest ausgewählt aus der Gruppe bestehend aus
-OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann;
wobei die Reste R⁷, R⁸ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und wobei, wenn p⁹ = 1, ―NR³R⁴ auch ein Rest der chemischen Struktur (x) sein kann,
und wobei, wenn p¹⁰ = 1, ―NR¹¹R¹² auch ein Rest der chemischen Struktur (x) sein kann,
und wobei die Reste ―NR⁵R⁶, ―NR⁷R⁸, ―NR⁹R¹⁰ unabhängig voneinander jeweils auch ein Rest der chemischen Struktur (x) sein können,
wobei die chemische Struktur (x) wie folgt definiert ist: 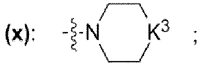
wobei K³ ausgewählt ist aus der Gruppe bestehend aus -O-, -S-, -NH-, -N(CH₃)-, -N(CH₂CH₃)-;
wobei die Reste R²¹, R²², R²³, R²⁴, R²⁵, R²⁶ unabhängig voneinander jeweils ausgewählt sind aus der Gruppe bestehend aus
Wasserstoff,
unverzweigte oder verzweigte Alkylgruppe mit 1 bis 30 Kohlenstoffatomen, einer Gruppe mit der chemischen Struktur (xi) mit 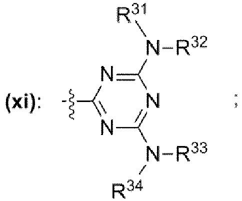
wobei die Reste R²⁷, R²⁸, R²⁹, R³¹, R³², R³³, R³⁴ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
unverzweigte oder verzweigte Acylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur ausgewählt aus der Gruppe bestehend aus **(xii), (xiii), (xiv), (xv), (xvi), (xvii), (xviii)** mit 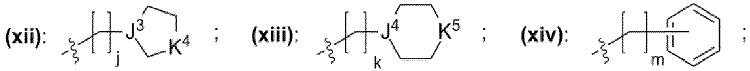 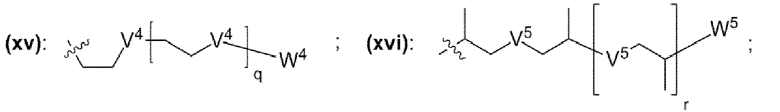 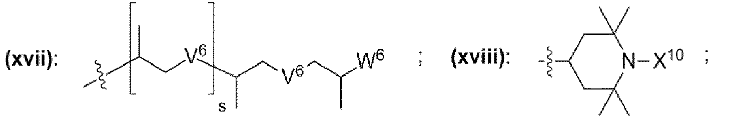 aufweist,
wobei J³, J⁴ unabhängig voneinander jeweils ausgewählt aus der Gruppe bestehend aus CH, N sind,
wobei K⁴, K⁵ unabhängig voneinander jeweils ausgewählt aus der Gruppe bestehend aus -O-, -NH-, -N(CH₃)-, -N(CH₂CH₃)-, -S-, -CH₂- sind,
wobei V⁴, V⁵, V⁶ unabhängig voneinander jeweils ausgewählt aus der Gruppe bestehend aus -O-, -S-, -NH-, -NR‴- mit R‴ = unverzweigte oder verzweigte Alkylgruppe mit 1 bis 6 Kohlenstoffatomen sind,
wobei W⁴, W⁵, W⁶ unabhängig voneinander jeweils ausgewählt aus der Gruppe bestehend aus H, Methyl, Ethyl sind,
wobei j, k, m, q, r, s unabhängig voneinander jeweils eine ganze Zahl aus dem Bereich 0 bis 50 ist,
wobei X¹⁰ = Wasserstoff,
wobei in den chemischen Strukturen **(xii), (xiii), (xiv), (xv), (xvi), (xvii), (xviii)** mindestens ein an ein Kohlenstoffatom gebundener Wasserstoffrest durch einen Rest ausgewählt aus der Gruppe bestehend
aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 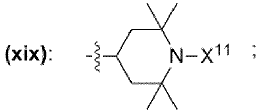 sein können, wobei X¹¹ = Wasserstoff,
und wobei man reduktive Bedingungen dadurch einstellt, dass man die mindestens eine Triacetonaminverbindung **(I)** mit Formaldehyd in Gegenwart von Wasserstoff und in Gegenwart eines Trägerkatalysators umsetzt, wobei der Trägerkatalysator mindestens ein Metall M aufweist, wobei das Metall **M** ausgewählt ist aus der Gruppe bestehend aus Cr, Mo, Mn, Ni, Pd, Pt, Ru, Rh.

2. Verfahren nach Anspruch 1, wobei p⁵ = p⁶ = p⁷ = p⁸ = p¹¹ = p¹² = p¹³ = p¹⁴ = 0 ist und wobei p⁹, p¹⁰, p¹⁵, p¹⁶, p¹⁷, p¹⁸, p¹⁹, p²⁰ unabhängig voneinander jeweils 0 oder 1 ist.

3. Verfahren nach Anspruch 1 oder 2, wobei Y³, Y⁴, Y⁵, Y⁶, Y⁷, Y⁸, Y⁹, Y¹⁰ unabhängig voneinander jeweils ausgewählt ist aus der Gruppe bestehend aus
unverzweigte oder verzweigte Alkylengruppe mit 1 bis 30 Kohlenstoffatomen,
zweiwertige gesättigte Kohlenwasserstoffgruppe mit 3 bis 30 Kohlenstoffatomen, welche mindestens einen gesättigten Ring aus 3 bis 30 Kohlenstoffatomen aufweist.

4. Verfahren nach einem oder mehreren der Ansprüche 1 bis 3, wobei die Reste R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(ix)** mit 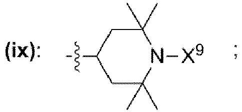 aufweist,
wobei X⁹ = Wasserstoff;
wobei die Reste R⁷, R⁸ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
und wobei, wenn p⁹ = 1, ―NR³R⁴ auch ein Rest der chemischen Struktur (x) sein kann,
und wobei, wenn p¹⁰ = 1, ―NR¹¹R¹² auch ein Rest der chemischen Struktur (x) sein kann,
und wobei die Reste ―NR⁵R⁶, ―NR⁷R⁸, ―NR⁹R¹⁰ unabhängig voneinander jeweils auch ein Rest der chemischen Struktur **(x)** sein können,
wobei die chemische Struktur **(x)** wie folgt definiert ist: 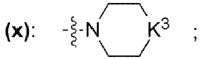 wobei K³ ausgewählt ist aus der Gruppe bestehend aus -O-, -S-, -NH-, -N(CH₃)-, -N(CH₂CH₃)-;
wobei die Reste R²¹, R²², R²³, R²⁴, R²⁵, R²⁶ unabhängig voneinander jeweils ausgewählt sind aus der Gruppe bestehend aus
Wasserstoff,
unverzweigte oder verzweigte Alkylgruppe mit 1 bis 30 Kohlenstoffatomen,
einer Gruppe mit der chemischen Struktur **(xi)** mit 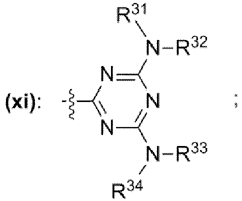
wobei die Reste R²⁷, R²⁸, R²⁹, R³¹, R³², R³³, R³⁴ unabhängig voneinander jeweils ausgewählt sind aus der Gruppe bestehend aus
Wasserstoff,
unverzweigte oder verzweigte Alkylgruppe mit 1 bis 30 Kohlenstoffatomen, bei welcher mindestens ein Wasserstoffrest durch einen Rest ausgewählt aus der Gruppe bestehend aus -OH, -NH₂, -OCH₃, -OCH₂CH₃, -NH(CH₃), -N(CH₃)₂, -NH(CH₂CH₃), -N(CH₂CH₃)₂, -N(CH₃)(CH₂CH₃) ersetzt sein kann,
einem Rest, der eine chemischen Struktur **(xviii)** mit 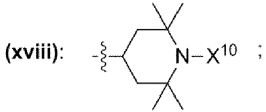
aufweist,
wobei X¹⁰ = Wasserstoff,
und mit der Maßgabe, dass R²¹ und R²⁶ für p¹⁷ = p¹⁸ = p¹⁹ = p²⁰ = 0 unabhängig voneinander jeweils auch eine Gruppe der chemischen Struktur **(xix)** mit 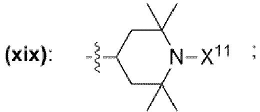 sein können, wobei X¹¹ = Wasserstoff.

5. Verfahren nach einem oder mehreren der Ansprüche 1 bis 4, wobei Formaldehyd als Gas, als wässrige Lösung oder als Feststoff eingesetzt wird.

6. Verfahren nach einem oder mehreren der Ansprüche 1 bis 5, wobei man die mindestens eine Triacetonaminverbindung **(I)** mit Formaldehyd unter reduktiven Bedingungen in mindestens einem Lösungsmittel umsetzt, wobei das Lösungsmittel ausgewählt ist aus der Gruppe bestehend aus aliphatische Lösungsmittel, aromatische Lösungsmittel, Ether, halogenierte Lösungsmittel, Amide, Thioverbindungen, Carbonsäuren, Alkohole, Wasser.

7. Verfahren nach einem oder mehreren der Ansprüche 1 bis 6, wobei man die mindestens eine Triacetonaminverbindung **(I)** mit Formaldehyd unter reduktiven Bedingungen bei einer Temperatur im Bereich von 20 °C bis 350 °C und einem Druck im Bereich von 2 bar bis 500 bar umsetzt.

## Claims

1. Process for preparing an *N*-methyl-substituted triacetonamine compound,
**characterized in that** at least one triacetonamine compound **(I)** is reacted with formaldehyde under reductive conditions,
where the triacetonamine compound **(I)** is selected from the group consisting of the chemical structures **(I-F), (I-G), (I-H)** with 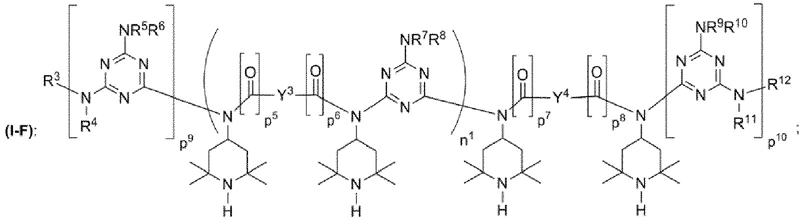 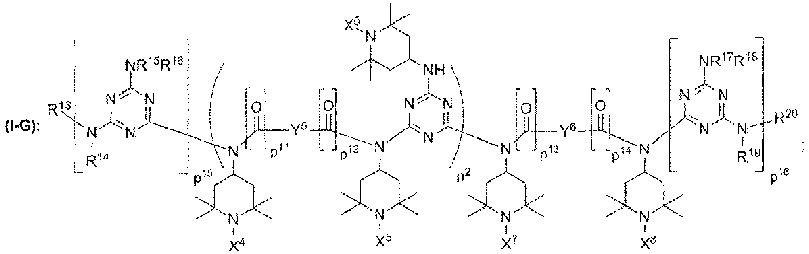 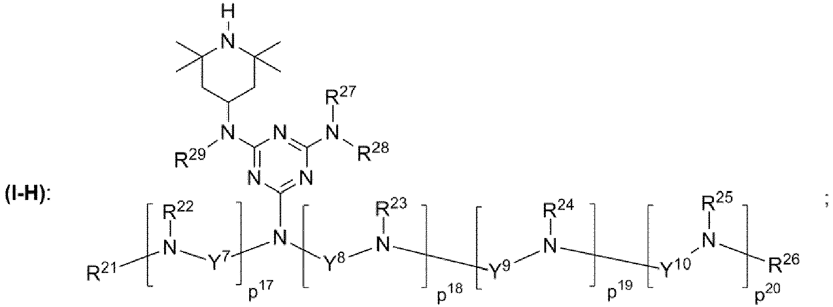
where n¹, n² are each independently an integer from the range of 1 to 20;
where p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰ are each independently 0 or 1;
where X⁴ = X⁵ = X⁶ = X⁷ = X⁸ = hydrogen;
where Y³, Y⁴, Y⁵, Y⁶, Y⁷, Y⁸, Y⁹, Y¹⁰ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms,
divalent hydrocarbyl group having 6 to 30 carbon atoms, of which at least 6 carbon atoms are present in an aromatic system and the other carbon atoms, if present, are saturated,
a bridging radical having a chemical structure selected from the group consisting of (i), (ii) with 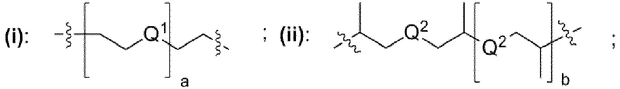
where
Q¹, Q² are each independently selected from the group consisting of -O-, -S-, -NH- and -NR'-with R' = unbranched or branched alkyl group having 1 to 6 carbon atoms,
where a is an integer selected from the range of 1 to 50,
where b is an integer selected from the range of 0 to 50,
and where Y³ may also be a direct bond if at least one of p⁵ and p⁶ has the value of 1,
and where Y⁴ may also be a direct bond if at least one of p⁷ and p⁸ has the value of 1,
and where Y⁵ may also be a direct bond if at least one of p¹¹ and p¹² has the value of 1,
and where Y⁶ may also be a direct bond if at least one of p¹³ and p¹⁴ has the value of 1;
where the R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
a radical having a chemical structure selected from the group consisting of **(iii), (iv), (v), (vi), (vii), (viii), (ix)** with 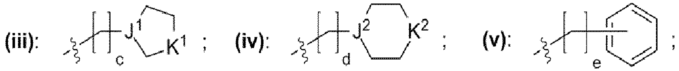 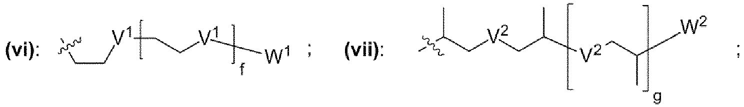 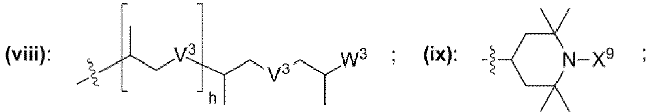
where J¹, J² are each independently selected from the group consisting of CH, N,
where K¹, K² are each independently selected from the group consisting of -O-, -NH-, -N(CH₃)-, -N(CH₂CH₃)-, -S-, -CH₂-,
where V¹, V², V³ are each independently selected from the group consisting of -O-, -S-, -NH-, - NR"- with R" = unbranched or branched alkyl group having 1 to 6 carbon atoms,
where W¹, W², W³ are each independently selected from the group consisting of H, methyl, ethyl, where c, d, e, f, g, h are each independently an integer from the range of 0 to 50,
where X⁹ = hydrogen,
where, in the chemical structures **(iii), (iv), (v), (vi), (vii), (viii), (ix),** at least one hydrogen radical bonded to a carbon atom may be replaced by a radical selected from the group consisting of -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃);
where the R⁷, R⁸ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
and where, when p⁹ = 1, -NR³R⁴ may also be a radical of the chemical structure **(x),**
and where, when p¹⁰ = 1, -NR¹¹R¹² may also be a radical of the chemical structure **(x),**
and where the -NR⁵R⁶, -NR⁷R⁸, -NR⁹R¹⁰ radicals may each independently also be a radical of the chemical structure **(x)**,
where the chemical structure **(x)** is defined as follows: **(x):** 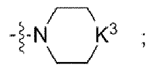
where K³ is selected from the group consisting of -O-, - S-, -NH-, -N(CH₃)-, -N(CH₂CH₃)-;
where the R²¹, R²², R²³, R²⁴, R²⁵, R²⁶ radicals are each independently selected from the group consisting of
hydrogen,
unbranched or branched alkyl group having 1 to 30 carbon atoms,
a group having the chemical structure **(xi)** with 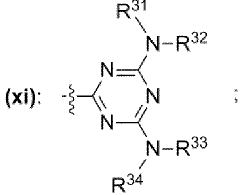
where the R²⁷, R²⁸, R²⁹, R³¹, R³², R³³, R³⁴ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
unbranched or branched acyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
a radical having a chemical structure selected from the group consisting of **(xii), (xiii), (xiv), (xv), (xvi), (xvii), (xviii)** with 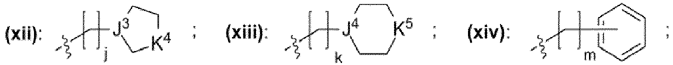 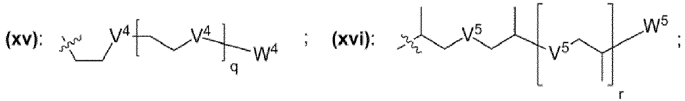 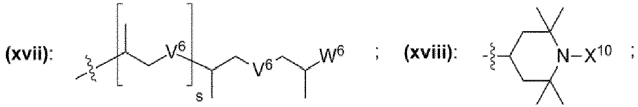
where J³, J⁴ are each independently selected from the group consisting of CH, N,
where K⁴, K⁵ are each independently selected from the group consisting of -O-, -NH-, -N(CH₃)-, -N(CH₂CH₃)-, -S-, -CH₂-,
where V⁴, V⁵, V⁶ are each independently selected from the group consisting of -O-, -S-, -NH-, - NR‴- with R‴ = unbranched or branched alkyl group having 1 to 6 carbon atoms,
where W⁴, W⁵, W⁶ are each independently selected from the group consisting of H, methyl, ethyl, where j, k, m, q, r, s are each independently an integer from the range of 0 to 50,
where X¹⁰ = hydrogen,
where, in the chemical structures **(xii), (xiii), (xiv), (xv), (xvi), (xvii), (xviii),** at least one hydrogen radical bonded to a carbon atom may be replaced by a radical selected from the group consisting of -OH, - NH₂, -OCH₃, -OCH₂CH₃, -NH(CH₃), -N(CH₃)₂,-NH(CH₂CH₃), -N(CH₂CH₃)₂, -N(CH₃)(CH₂CH₃),
and with the proviso that R²¹ and R²⁶, when p¹⁷ = p¹⁸ = p¹⁹ = p²⁰ = 0, may each independently also be a group of the chemical structure **(xix)** with 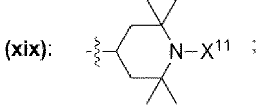
where X¹¹ = hydrogen,
and where reductive conditions are established by reacting the at least one triacetonamine compound **(I)** with formaldehyde in the presence of hydrogen and in the presence of a supported catalyst, where the supported catalyst includes at least one metal **M,** where the metal **M** is selected from the group consisting of Cr, Mo, Mn, Ni, Pd, Pt, Ru, Rh.

2. Process according to Claim 1, where p⁵ = p⁶ = p⁷ = p⁸ = p¹¹ = p¹² = p¹³ = p¹⁴ = 0 and where p⁹, p¹⁰, p¹⁵, p¹⁶, p¹⁷, p¹⁸, p¹⁹, p²⁰ are each independently 0 or 1.

3. Process according to Claim 1 or 2, where Y³, Y⁴, Y⁵, Y⁶, Y⁷, Y⁸, Y⁹, Y¹⁰ are each independently selected from the group consisting of
unbranched or branched alkylene group having 1 to 30 carbon atoms,
divalent saturated hydrocarbyl group having 3 to 30 carbon atoms and having at least one saturated ring composed of 3 to 30 carbon atoms.

4. Process according to one or more of Claims 1 to 3, where the R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
a radical having a chemical structure **(ix)** with 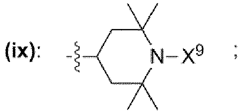 where X⁹ = hydrogen;
where the R⁷, R⁸ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
and where, when p⁹ = 1, -NR³R⁴ may also be a radical of the chemical structure **(x),**
and where, when p¹⁰ = 1, -NR¹¹R¹² may also be a radical of the chemical structure **(x),**
and where the -NR⁵R⁶, -NR⁷R⁸, -NR⁹R¹⁰ radicals may each independently also be a radical of the chemical structure **(x)**,
where the chemical structure **(x)** is defined as follows: 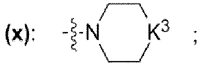
where K³ is selected from the group consisting of -O-, - S-, -NH-, -N(CH₃)-, -N(CH₂CH₃)-;
where the R²¹, R²², R²³, R²⁴, R²⁵, R²⁶ radicals are each independently selected from the group consisting of
hydrogen,
unbranched or branched alkyl group having 1 to 30 carbon atoms,
a group having the chemical structure **(xi)** with 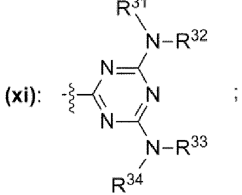
where the R²⁷, R²⁸, R²⁹, R³¹, R³², R³³, R³⁴ radicals are each independently selected from the group consisting of
hydrogen,
unbranched or branched alkyl group which has 1 to 30 carbon atoms and in which at least one hydrogen radical may be replaced by a radical selected from the group consisting of -OH, -NH₂, -OCH₃, -OCH₂CH₃, -NH(CH₃), -N(CH₃)₂, -NH(CH₂CH₃), -N(CH₂CH₃)₂,-N(CH₃)(CH₂CH₃),
a radical having a chemical structure **(xviii)** with 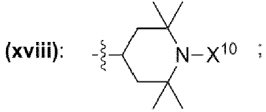
where X¹⁰ = hydrogen,
and with the proviso that R²¹ and R²⁶, when p¹⁷ = p¹⁸ = p¹⁹ = p²⁰ = 0, may each independently also be a group of the chemical structure **(xix)** with 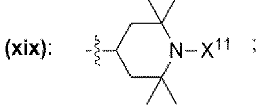 where X¹¹ = hydrogen.

5. Process according to one or more of Claims 1 to 4, where formaldehyde is used as a gas, as an aqueous solution or as a solid.

6. Process according to one or more of Claims 1 to 5, where the at least one triacetonamine compound **(I)** is reacted with formaldehyde under reductive conditions in at least one solvent, where the solvent is selected from the group consisting of aliphatic solvents, aromatic solvents, ethers, halogenated solvents, amides, thio compounds, carboxylic acids, alcohols, water.

7. Process according to one or more of Claims 1 to 6, where the at least one triacetonamine compound **(I)** is reacted with formaldehyde under reductive conditions at a temperature in the range from 20°C to 350°C and a pressure in the range from 2 bar to 500 bar.

## Revendications

1. Procédé pour la préparation d'un composé de type triacétonamine substitué par N-méthyle,
**caractérisé en ce qu'**on transforme au moins un composé de type triacétonamine (I) avec du formaldéhyde dans des conditions réductrices,
où le composé de type triacétonamine (I) est choisi dans le groupe constitué par les structures chimiques (I-F), (I-G), (I-H) : 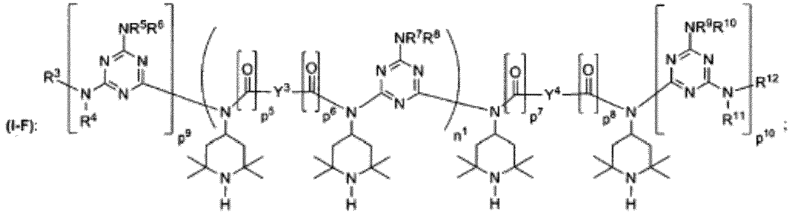 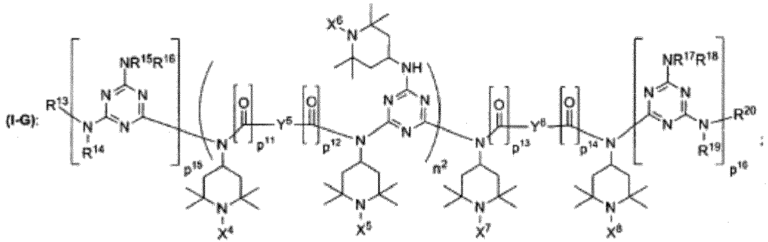 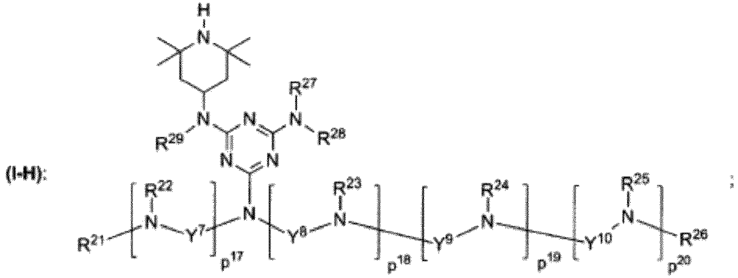
où n¹, n², indépendamment l'un de l'autre, sont à chaque fois un nombre entier dans la plage de 1 à 20 ;
où p⁵, p⁶, p⁷, p⁸, p⁹, p¹⁰, p¹¹, p¹², p¹³, p¹⁴, p¹⁵, p¹⁶, p¹⁷, p¹⁸, p¹⁹, p²⁰, indépendamment les uns des autres, valent à chaque fois 0 ou 1 ;
où X⁴ = X⁵ = X⁶ = X⁷ = X⁸ = hydrogène ;
où Y³, Y⁴, Y⁵, Y⁶, Y⁷, Y⁸, Y⁹, Y¹⁰, indépendamment les uns des autres, sont à chaque fois choisis dans le groupe constitué par
groupe alkylène non ramifié ou ramifié comprenant 1 à 30 atomes de carbone,
groupe hydrocarboné saturé bivalent comprenant 3 à 30 atomes de carbone, qui présente au moins un cycle saturé constitué par 3 à 30 atomes de carbone, groupe hydrocarboné bivalent comprenant 6 à 30 atomes de carbone, dont au moins 6 atomes de carbone se trouvent dans un système aromatique et dont les autres atomes de carbone, s'ils sont présents, sont saturés,
un radical formant un pont, qui présente une structure chimique choisie dans le groupe constitué par (i), (ii) : 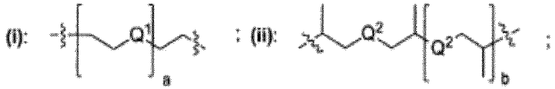
où Q¹, Q², indépendamment l'un de l'autre, sont à chaque fois choisis dans le groupe constitué par -O-, -S-, -NH- ou -NR'- où R' = groupe alkyle non ramifié ou ramifié comprenant 1 à 6 atomes de carbone,
où a est un nombre entier choisi dans la plage de 1 à 50,
où b est un nombre entier choisi dans la plage de 0 à 50,
et où Y³ peut également être une liaison directe, si au moins l'un parmi p⁵ et p⁶ vaut 1,
et où Y⁴ peut également être une liaison directe, si au moins l'un parmi p⁷ et p⁸ vaut 1,
et où Y⁵ peut également être une liaison directe, si au moins l'un parmi p¹¹ et p¹² vaut 1,
et où Y⁶ peut également être une liaison directe, si au moins l'un parmi p¹³ et p¹⁴ vaut 1,
où les radicaux R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, indépendamment les uns des autres, sont à chaque fois choisis dans le groupe constitué par
hydrogène,
groupe alkyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
groupe acyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
un radical, qui présente une structure chimique choisie dans le groupe constitué par (iii), (iv), (v), (vi), (vii), (viii), (ix) : 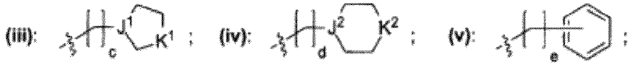 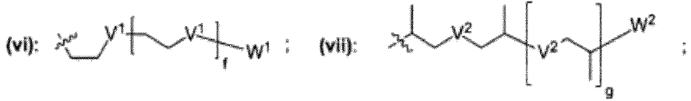 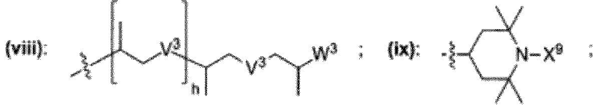
où J¹ et J², à chaque fois indépendamment l'un de l'autre, sont à chaque fois choisis dans le groupe constitué par CH, N,
où K¹, K², indépendamment l'un de l'autre, sont à chaque fois choisis dans le groupe constitué par -O-, -NH-, -N(CH₃)-, -N(CH₂CH₃)-, -S-, -CH₂-,
où V¹, V², V³, indépendamment les uns des autres, sont à chaque fois choisis dans le groupe constitué par -O-, -S-, -NH-, -NR"- où R" = groupe alkyle non ramifié ou ramifié comprenant 1 à 6 atomes de carbone,
où W¹, W², W³, indépendamment les uns des autres, sont à chaque fois choisis dans le groupe constitué par H, méthyle, éthyle,
où c, d, e, f, g, h, indépendamment les uns des autres, représentent à chaque fois un nombre entier dans la plage de 0 à 50,
où X⁹ = hydrogène,
où, dans les structures chimiques (iii), (iv), (v), (vi), (vii), (viii), (ix), au moins un radical hydrogène lié à un atome de carbone peut être remplacé par un radical choisi dans le groupe constitué par -OH, -NH₂, -OCH₃, -OCH₂CH₃, -NH(CH₃), - N(CH₃)₂, -NH(CH₂CH₃), -N(CH₂CH₃)₂, -N(CH₃)(CH₂CH₃) ;
où les radicaux R⁷, R⁸, indépendamment l'un de l'autre, sont à chaque fois choisis dans le groupe constitué par
hydrogène,
groupe alkyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
groupe acyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
et où, lorsque p⁹ = 1, -NR³R⁴ peut également être un radical de structure chimique (x),
et où, lorsque p¹⁰ = 1, -NR¹¹R¹² peut également être un radical de structure chimique (x),
et où les radicaux -NR⁵R⁶, -NR⁷R⁸, -NR⁹R¹⁰, indépendamment les uns des autres, peuvent à chaque fois également être un radical de structure chimique (x),
où la structure chimique (x) est définie comme suit : **(x):** 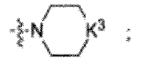
où K³ est choisi dans le groupe constitué par -O-, -S-, -NH-, -N(CH₃)-, -N(CH₂CH₃)- ;
où les radicaux R²¹, R²², R²³, R²⁴, R²⁵, R²⁶, indépendamment les uns des autres, sont à chaque fois choisis dans le groupe constitué par :
hydrogène,
groupe alkyle non ramifié ou ramifié comprenant 1 à 30 atomes de carbone,
un groupe présentant la structure chimique (xi) : 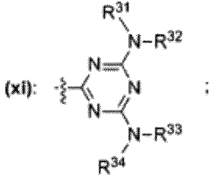
où les radicaux R²⁷, R²⁸, R²⁹, R³¹, R³², R³³, R³⁴, indépendamment les uns des autres, sont à chaque fois choisis dans le groupe constitué par :
hydrogène,
groupe alkyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
groupe acyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
un radical, qui présente une structure chimique choisie dans le groupe constitué par (xii), (xiii), (xiv), (xv), (xvi), (xvii), (xviii) : 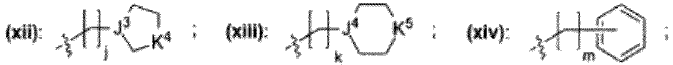 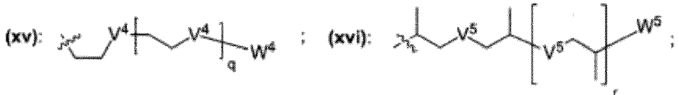 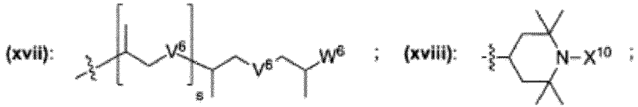
où J³ et J⁴, indépendamment l'un de l'autre, sont à chaque fois choisis dans le groupe constitué par CH, N,
où K⁴, K⁵, indépendamment l'un de l'autre, sont à chaque fois choisis dans le groupe constitué par -O-, -NH-, -N(CH₃)-, -N(CH₂CH₃)-, -S-, -CH₂-,
où V⁴, V⁵, V⁶, indépendamment les uns des autres, sont à chaque fois choisis dans le groupe constitué par -O-, -S-, -NH-, -NR‴- où R‴ = groupe alkyle non ramifié ou ramifié comprenant 1 à 6 atomes de carbone,
où W⁴, W⁵, W⁶, indépendamment les uns des autres, sont à chaque fois choisis dans le groupe constitué par H, méthyle, éthyle,
où j, k, m, q, r, s, indépendamment les uns des autres, représentent à chaque fois un nombre entier dans la plage de 0 à 50 ;
où X¹⁰ = hydrogène,
où, dans les structures chimiques (xii), (xiii), (xiv), (xv), (xvi), (xvii), (xviii), au moins un radical hydrogène lié à un atome de carbone peut être remplacé par un radical choisi dans le groupe constitué par -OH, -NH₂, -OCH₃, -OCH₂CH₃, -NH(CH₃), -N(CH₃)₂, -NH(CH₂CH₃), -N(CH₂CH₃)₂, - N(CH₃)(CH₂CH₃);
et à condition que R²¹ et R²⁶, pour p¹⁷ = p¹⁸ = p¹⁹ = p²⁰ = 0, indépendamment l'un de l'autre, puissent à chaque fois également représenter un groupe de structure chimique (xix) : 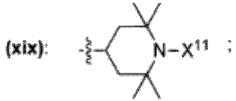
où X¹¹ = hydrogène,
et où des conditions réductrices sont réglées **en ce que** ledit au moins un composé de type triacétonamine (I) est transformé avec du formaldéhyde en présence d'hydrogène et en présence d'un catalyseur supporté, le catalyseur supporté présentant au moins un métal M, le métal M étant choisi dans le groupe constitué par Cr, Mo, Mn, Ni, Pd, Pt, Ru, Rh.

2. Procédé selon la revendication 1, où p⁵ = p⁶ = p⁷ = p⁸ = p¹¹ = p¹² = p¹³ = p¹⁴ = 0 et p⁹, p¹⁰, p¹⁵, p¹⁶, p¹⁷, p¹⁸, p¹⁹, p²⁰, indépendamment les uns des autres, valent à chaque fois 0 ou 1.

3. Procédé selon la revendication 1 ou 2, où Y³, Y⁴, Y⁵, Y⁶, Y⁷, Y⁸, Y⁹, Y¹⁰, indépendamment les uns des autres, sont à chaque fois choisis dans le groupe constitué par
groupe alkylène non ramifié ou ramifié comprenant 1 à 30 atomes de carbone,
groupe hydrocarboné saturé bivalent comprenant 3 à 30 atomes de carbone, qui présente au moins un cycle saturé constitué par 3 à 30 atomes de carbone.

4. Procédé selon l'une ou plusieurs des revendications 1 à 3, où les radicaux R³, R⁴, R⁵, R⁶, R⁹, R¹⁰, R¹¹, R¹², R¹³, R¹⁴, R¹⁵, R¹⁶, R¹⁷, R¹⁸, R¹⁹, R²⁰, indépendamment les uns des autres, sont à chaque fois choisis dans le groupe constitué par
hydrogène,
groupe alkyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
un radical qui présente une structure chimique (ix) : 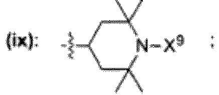
où X⁹ = hydrogène
où les radicaux R⁷, R⁸, indépendamment l'un de l'autre, sont à chaque fois choisis dans le groupe constitué par
hydrogène,
groupe alkyle non ramifié ou ramifié comprenant 1 à 30 atomes de carbone, dans lequel au moins un radical hydrogène peut être remplacé par un radical choisi dans le groupe constitué par -OH, -NH₂, - OCH₃, -OCH₂CH₃, -NH(CH₃), -N(CH₃)₂, -NH(CH₂CH₃), - N(CH₂CH₃)₂, -N(CH₃)(CH₂CH₃),
et où, lorsque p⁹ = 1, -NR³R⁴ peut également être un radical de structure chimique (x),
et où, lorsque p¹⁰ = 1, -NR¹¹R¹² peut également être un radical de structure chimique (x),
et où les radicaux -NR⁵R⁶, -NR⁷R⁸, -NR⁹R¹⁰, indépendamment les uns des autres, peuvent à chaque fois également être un radical de structure chimique (x),
où la structure chimique (x) est définie comme suit : 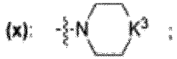
où K³ est choisi dans le groupe constitué par -O-, -S-, -NH-, -N(CH₃)-, -N(CH₂CH₃)- ;
où les radicaux R²¹, R²², R²³, R²⁴, R²⁵, R²⁶, indépendamment les uns des autres, sont à chaque fois choisis dans le groupe constitué par :
hydrogène,
groupe alkyle non ramifié ou ramifié comprenant 1 à 30 atomes de carbone,
un groupe présentant la structure chimique (xi) 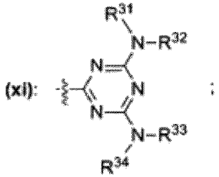
où les radicaux R²⁷, R²⁸, R²⁹, R³¹, R³², R³³, R³⁴, indépendamment les uns des autres, sont à chaque fois choisis dans le groupe constitué par :
hydrogène,
groupe alkyle non ramifié ou ramifié comprenant 1 à 30 atomes de carbone, dans lequel au moins un radical hydrogène peut être remplacé par un radical choisi dans le groupe constitué par ―OH, ―NH₂, ― OCH₃, ―OCH₂CH₃, ―NH(CH₃), ―N(CH₃)₂, ―NH(CH₂CH₃), ― N(CH₂CH₃)₂, ―N(CH₃)(CH₂CH₃),
un radical qui présente une structure chimique (xviii) 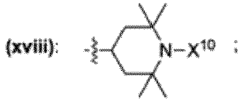
où X¹⁰ = hydrogène,
et à condition que R²¹ et R²⁶, pour p¹⁷ = p¹⁸ = p¹⁹ = p²⁰ = 0, indépendamment l'un de l'autre, puissent à chaque fois également représenter un groupe de structure chimique (xix) : 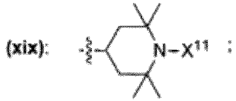 où X¹¹ = hydrogène.

5. Procédé selon l'une ou plusieurs des revendications 1 à 4, où le formaldéhyde est utilisé sous forme de gaz, de solution aqueuse ou de solide.

6. Procédé selon l'une ou plusieurs des revendications 1 à 5, où l'au moins un composé de type triacétonamine (I) est transformé avec du formaldéhyde dans des conditions réductrices dans au moins un solvant, le solvant étant choisi dans le groupe constitué par les solvants aliphatiques, les solvants aromatiques, les éthers, les solvants halogénés, les amides, les composés thio, les acides carboxyliques, les alcools, l'eau.

7. Procédé selon l'une ou plusieurs des revendications 1 à 6, où l'au moins un composé de type triacétonamine (I) est transformé avec du formaldéhyde dans des conditions réductrices à une température dans la plage de 20°C à 350°C et à une pression dans la plage de 2 bars à 500 bars.
